# EUROPEAN PATENT SPECIFICATION

(11) **EP 1 649 813 B1**
(45) Date of publication and mention of the grant of the patent: **13.04.2016**
(21) Application number: 04770978.7
(22) Date of filing: 28.07.2004
(51) Int. Cl.: A61B 5/16

(54) **DEMENTIA CHECK APPARATUS**
VORRICHTUNG ZUR ÜBERPRÜFUNG VON DEMENZ
DISPOSITIF DE VERIFICATION DE LA DEMENCE

(30) Priority: 28.07.2003 JP 2003280626
(43) Date of publication of application: 26.04.2006
(73) Proprietor: Sosei Ltd., Naka-ku, Hamamatsu-shi, Shizuoka 432-8002 (JP)
(72) Inventor: SHIMURA, Takaki, Tokai University, Hiratsuka-shi, Kanagawa 259-1207 (JP); OZAKI, Masako, Hadano-shi Kanagawa 257-0003 (JP); KANEKO, Mitsuo, Hamamatsu-shi, Shizuoka 433-8123 (JP)
(74) Representative: Fenlon, Christine Lesley
(86) International application number: PCT/JP2004/010715
(87) International publication number: WO 2005/009247

(56) References cited:
- JP-A- 2002 143 096
- JP-A- 2003 073 631
- US-A1- 2002 059 031
- US-A1- 2003 059 759
- KOSS E ET AL: "THE STROOP COLOR-WORD TEST: INDICATOR OF DEMENTIA SEVERITY" INTERNATIONAL JOURNAL OF NEUROSCIENCE, GORDON AND BREACH, US, vol. 24, no. 1, 1 January 1984 (1984-01-01), pages 53-61, XP008022167 ISSN: 0020-7454

## Description

### TECHNICAL FIELD

The present invention relates to a dementia inspection apparatus.

### BACKGROUND ART

We are in the 21st century, and a situation in which the graying and birth dearth proceed at the same time in Japanese society will continue. The number of senior citizens who need to have care is increased, and the number of age brackets who care such senior citizens is reduced. In such a situation, the care is a nationalistic important problem concerning health of senior citizens.

Among them, the senile dementia is difficult problem to be solved because if the senile dementia proceeds to serious degree, it is impossible to recover, and large labor is required for taking care such person. Recent research makes it apparent that if a dementia patient carries out rehabilitation or training at relatively early or light stage, it is possible to recover or it is effective as safeguard. In view of such background, it can sufficiently be expected that the needs for ready detection of senile dementia is increased.

A frontal lobe of a brain controls the judgment function of a human, dementia can be classified variously depending upon the generation causes, and it is said that the most polular senile dementia (or obsolete dementia) is caused by funciton deterioration of the frontal lobe. It is known that a test which simultaneously requires several judgments is effective for inspecting the judgment made by the frontal lobe function.

As one technique concerning the ready detection of the senile dementia, a dementia inspection apparatus, a dementia inspection system, a dementia inspection server and a dementia inspection client constituting the dementia inspection system which uses a dementia degree inspection chart requiring an answer in a style in which several kinds of judgments are required at the same time and correct or error can be determined objectively (e.g., see JP-A No.2002-143096).

### DISCLOSURE OF THE INVENTION

As one example of the dementia degree examining chart, the technique shown in Japanese Patent Application Laid-open (JP-A) No.2002-143096 shows a dementia degree examining chart which corresponds both kanji color discerning test and kana reading test. Here, the kanji color discerning test is a test in which a list of kanjis respectively tinted with different colors and representing colors are shown to a subject, and the subject is allowed to determine whether the kanji and its color are matched or mismatched. The kana reading test is a test in which a subject is allowed read sentences all written in hiragana only, and the subject is allowed to simultaneously carry out two operations, i. e, an operation for picking up kana characters representing the sound of "a", "i", "u", "e" and "o" which are previously determined in the sentences, and an operation for understanding and memorizing contents of each sentence.

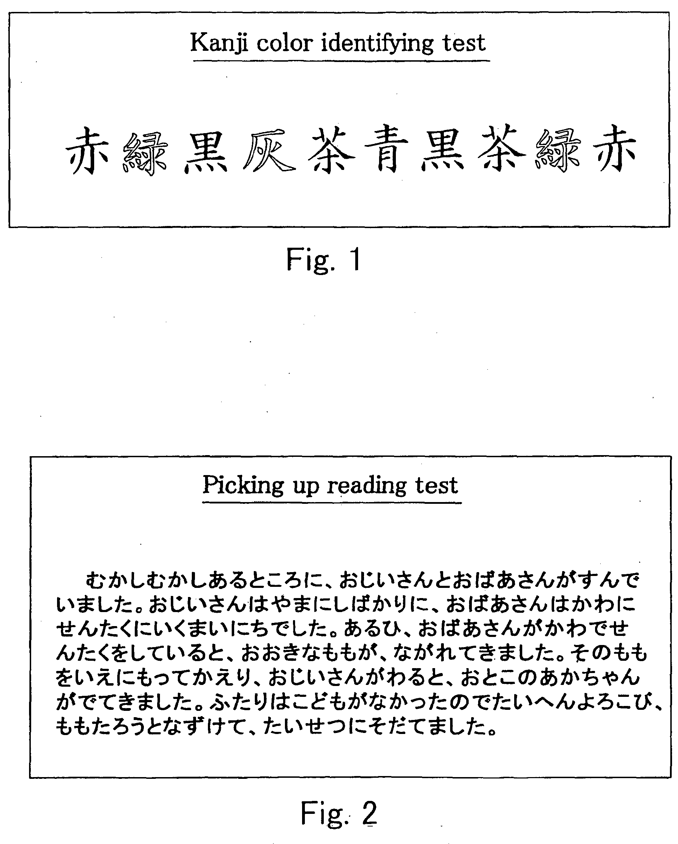

Fig. 1 shows one example of the dementia degree examining chart used in the kanji color discerning test, and Fig. 2 shows one example of the dementia degree examining chart used in the kana reading test.

Here, in the kanji color discerning test, distribution ability of one attention for judging whether the color meant by a kanji and the printed color of the kanji are matched or mismatched is evaluated. If the kanji color discerning test is compared with the kana reading test in which the distributing ability of two attentions for picking up kana characters from the indicated sentences and understanding and memorizing the contents of the sentences, there is a problem that the kanji color discerning test has lower difficulty level, and sevior evaluation result can not be obtained.

On the other hand, the kana reading test is a test which is inherent in Japanese language, and it is difficult to employ this test as an international test. For example, a test similar to the kana reading test is to be carried out, a subj ect is allowed to carry out an operation for picking up predetermined characters such as alphabet from English sentences. It is known that the attention distribution ability which is exhibited by Japanese required for picking characters from Japanese sentences, and the attention distribution ability which is exhibited by English native speaker required for picking alphabet from predetermined English sentences are look like similar but actually very different from each other. That is, in such a test, it is difficult to obtain internationally common evaluation result.

The present invention has been accomplished in view of the above circumstances, and it is an object of the invention to provide a dementia inspection apparatus which is internationally effective for ready detection of initial symptom of senile dementia.

To achieve the above obj ect, the present inventionprovides a dementia inspection apparatus comprising: an answer obtaining section; and a dementia degree inspecting section,
wherein the answer obtaining section obtains answers from a subject made within a predetermined answer time limit to each of a first examination chart and a second examination chart, the first examination chart has inspection sentences in which a character group constituting a story including color words each representing color is tinted with plural colors such that individual color word has characters of the same color, requires a determination as to whether a color of characters constituting the color word is the same color as color represented by the color word, and requires an answer in a style capable of objectively determining whether the determination is correct or error, the second examination chart has a combination of plural questions concerning contents of the inspection sentences and plural answers which are prepared for each question and one of which is to be selected, and
the dementia degree inspection section determines a dementia degree representing a degree of dementia of the subject based on the answers obtained by the answer obtaining section.

According to the present invention, it is possible to obtain evaluation with respect to the attention distributing ability required to pick up color words representing colors from the inspection sentences by the first examination chart, and evaluation with respect to the attention distributing ability required to determine whether color represented by a color word and color of the color word match with each other or not. Further, it is possible to obtain evaluation with respect to the attention distributing ability required to understand and memorize the contents of the inspection sentences by the second examination chart. With this, it is possible to carry out a test having high difficulty level as compared with the above-described word color discerning test.

The idea of the first examination chart can be applied to Japanese language of course, but also can be applied to other language such as English.

For example, when the first examination chart is applied to Japanese language, it is required to determine whether color of a color kanj i in an inspection sentence including color kanj is representing colors and a color represented by that color kanji are the same, a chart is prepared in which answer is required in such a style that correct or error of the determination can be found objectively, thereby carrying out the test. When the first examination chart is applied to English, it is required to determine whether color of a color word in the inspection sentence including color word representing color is the same as color represented by the color word, a chart is prepared in which answer is required in such a style that correct or error of the determination can be found objectively, thereby carrying out the test. In this manner, even if plural subjects whose mother languages are different from each other are to receive the inspections, if first examination charts whose color words in the respective mother languages are colored are prepared, the subjects can be required to exhibit the same determining abilities. Thus, according to the first examination chart, it is possible to carry out the internationally general inspection of dementia degree of subject.

Here, in the dementia inspection apparatus of the present invention, it is preferable that the apparatus further comprises a first examination chart forming section, wherein the first examination chart forming section has plural kinds of inspection sentences tinted with colors in different coloring manners and selects one kind of inspection sentences from the plural kinds of inspection sentences as inspection sentences of the first examination chart, thereby forming the first examination chart.

In the dementia inspection apparatus of the invention, it can be assumed that the same subject receives the test repeatedly. At that time, if the same subject repeatedly uses the same examination chart, there is a repetition learning effect in which the subject learns answers of the examination chart. Hence, according to the dementia inspection apparatus having the first examination chart forming section, for the same subject, a first examination chart which is different from that used in the last inspection is produced, thereby suppressing the repetition learning effect.

In the dementia inspection apparatus, it is preferable that the apparatus further comprises comprising a second examination chart forming section, the second examination chart forming section has plural kinds of question units each comprising a combination of a question concerning a story represented by the inspection sentences and plural answers which are prepared for the question and which are to be selected, a predetermined number of question units are selected from the plural kinds of question units, thereby forming the second examination chart.

If the apparatus has such a second examination chart forming section, it is possible to suppress the repetition learning effect of the subject with respect to the second examination chart.

In the dementia inspection apparatus having the second examination chart forming section, it is preferable that
the plural kinds of question units are associated with positions in the inspection sentences,
the dementia inspection apparatus further comprises a sentence range detecting section which detects a range of the inspection sentences which a subject was able to read within a predetermined answer time limit with respect to the first examination chart,
the second examination chart forming section selects a question unit associated with a position in the range detected by the sentence range detecting section in the inspection sentence, thereby forming the second examination chart.

For example, there is a possibility that a subject can not read off of the inspection sentences within in the predetermined answer time limit with respect to the first examination chart. In such a case, if a question concerning such a portion of the inspection sentences that the subject has not yet read is included in the second examination chart, since the subj ect does not known the contents concerning this question, the subject answers by pure guesswork which is not related to understanding or memory of the subject. There is an adverse possibility that answer having nothing to do with understanding or memory deteriorates the reliability of inspection result with respect to dementia degree of a subject. Hence, according to the dementia inspection apparatus of the preferred aspect, the sentence range detecting section detects a range in the inspection sentences which the subject finished reading, and only questions concerning the contents within the range is shown to the subject. Therefore, according to the dementia inspection apparatus of the preferred aspect, since it is possible to prevent meaningless answer of a subject, it is possible to obtain more reliable inspection result of the dementia degree of the subject.

In the dementia inspection apparatus of the present invention having the first examination chart producing section, the color words in the character group of the inspection sentences are tinted with plural colors such that individual color word has characters of the same color, characters in the character group except the color words are tinted with a single color.

If such an inspection sentence is used, for example, it is possible to produce a first examination chart having inspection sentences in which color words representing color in the sentences are tinted with plural colors, and other characters in the sentence are tinted with a single black color.

According to the dementia inspection apparatus of the invention, in the inspection sentences, character strings divided by specific kind of characters (e. g. punctuation, period or the like) are tinted with colors different from each other.

If such inspection sentences are used, it is possible to produce a first examination chart or the like having an inspection sentence in which character strings divided by commas in a sentence are tinted with different colors. If such a first examination chart is used, it is possible to carry out an inspection having higher difficulty level for a subject as compared with a case using a first examination chart having an inspection sentence in which only color words representing color are tinted with colors different from those of other characters in the sentence.

Further, in the dementia inspection apparatus, it is preferable that the apparatus comprises:
a chart display section which displays the first examination chart and the second examination chart;
a start instructing section which instructs the subject to start inputting answers to the first examination chart and the second examination chart; and
an answer time control section which prohibits the subj ect from inputting answer when time elapsed after the start instructing section instructed to start inputting answer to the first examination chart reaches a predetermined answer time limit in accordance with the first examination chart, and which prohibits the subject from inputting answer when time elapsed after the start instructing section instructed to start inputting answer to the second examination chart reaches a predetermined answer time limit in accordance with the second examination chart.

According to the start instructing section, it is possible to clearly indicate, for a subject, start of input of answers to the first examination chart and second examination chart. By setting time limit for inputting answers to the first examination chart and second examination chart by means of the answer time control section, it is possible to carry out inspection having higher difficulty level for a subject.

In the dementia inspection apparatus of the invention, it is preferable that the apparatus further comprises a result display section which displays a dementia degree inspection result obtained by the dementia degree inspecting section.

By providing the apparatus with the result display section, it is possible to immediately and easily confirm the dementia degree of the subject.

In the dementia inspection apparatus of the invention, it is preferable that the apparatus further comprises an inspection receiving permission judging section which permits or prohibits a request for inspection of an inspection wisher in accordance with whether or not a predetermined period of time is elapsed after the inspection wisher received inspection last time.

Since the apparatus has the inspection receiving permission judging section, the same subject does not receive the same test repeatedly, and it is possible to suppress the repetition learning effect.

In the dementia inspection apparatus, it is preferable that the apparatus further comprises a dementia degree storing sectionwhich stores correspondence between answers and dementia degree with respect to both the first examination chart and second examination chart, wherein
the dementia degree inspecting section refers to the dementia degree storing section, and determines a dementia degree of the subject from answers to both the first examination chart and second examination chart of the current subject obtained by the answer obtaining section.

The apparatus has the dementia degree storing section, and it is possible to objectively determine the dementia degree of the subject by referring to the correspondence between dementia degree and answers to both the first examination chart and second examination chart stored in the dementia degree storing section.

In the dementia inspection apparatus, from the answers to the first examination chart obtained by the answer obtaining section, the dementia degree inspecting section extracts a number of correct answers as to whether color of characters constituting the color word is the same color as that represented by the color word, a number of error answers erroneously determined, a number of oversights of color words, and a number of erroneous recognition of characters other than the color words, and extracts a number of correct meaning grasps with respect to plural questions concerning a story described by the inspection sentences from answers to the second examination chart obtained by the answer obtaining section, and compares these extracted numbers with predetermined reference values with respect to these numbers, thereby determining the dementia degree,
the dementia degree storing section stores the predetermined reference values.

Several evaluation items are set, reference values of the respective evaluation items are stored in the dementia degree storing section, and a result obtained from an answer from the subject and a reference value stored in the dementia degree storing section are compared with each other for each of evaluation items by the dementia degree inspecting section. With this, it is possible to determine the dementia degree of a subject objectively.

In the dementia inspection apparatus, in plural alternatives which are prepared as answers to the questions and one of which is to be selected, the second examination chart includes alternatives for informing that the subject does not know correct answer,
the dementia degree inspecting section further extracts a number of unclear answers showing how many alternatives for informing that the subject does not know correct answer the subject selected from answers to the second examination chart obtained by the answer obtaining section, and determines the dementia degree based on the number of unclear answers, the number of correct answers, the number of error answers, the number of oversights, the number of erroneous recognition, and the number of meaning grasps.

For example, when a subject answers to one question in the second examination chart, suppose that the subject does not memorize contents of a portion concerning the question among a story described by the inspection sentence. In such a case, if no alternative (e.g., no idea, forgot, or the like) for indicating that the subject has no idea is included in alternatives prepared as answers to the questions in the second examination chart, the subject answers by pure guesswork which is not related to understanding or memory of the subject. At that time, the subj ect can select a correct answer from the plural answers to the question by coincidence. If there exist correct meaningless answers which have nothing to do with understanding or memory of a subject, entity shown by the number of meaning grasps is incorrect, and reliability of inspection result with respect to the dementia degree of a subject is deteriorated. Hence, according to the dementia inspection apparatus of the preferred aspect, since the alternative for indicating that a subject has no idea is included in the alternatives, it is possible to prevent a subject from inputting meaningless correct answer. In the dementia degree inspecting section, determination is made to see a dementia degree using the number of unclear answers showing how many alternatives indicative of no idea were selected. Thus, it is possible to obtain more reliable inspection result.

It is preferable that the dementia inspection apparatus of the present invention includes a pre-test executing section which carries out a pre-test including a pre-test first examination chart and a pre-test second examination chart before the dementia degree is determined using the first examination chart and the second examination chart, the pre-test first examination chart includes pre-test sentences in which a character group constituting a story including color words each representing color is tinted with plural colors such that individual color word has characters of the same color, requires a determination as to whether a color of the characters constituting the color word is the same color as color represented by the color word, and requires an answer in a style capable of objectively determining whether the determination is correct or error, the pre-test second examination chart has a combination of plural questions concerning contents of the pre-test sentences and plural answers which are prepared for each question and one of which is to be selected,
wherein the answer obtaining section obtains answers of the subject made within predetermined answer time limits to both the pre-test first examination and the pre-test second examination chart, and
wherein the dementia degree inspecting section determines the dementia degree which represents a degree of dementia of the subj ect based on the answers to the pre-test first examination chart and the pre-test second examination chart.

Since the apparatus has the pre-test executing section, a subject can become used to the operation of the dementia inspection apparatus before the real inspection.

The basic idea of the present invention can be constituted as a server/client system utilizing the Internet for example. With this, more people can have chances to receive the inspections of dementia degrees. Here, a server and a client of the server/client system are called a dementia inspection server and a dementia inspection client.

As explained above, according to the present invention, it is possible to easily carry out an internationally effective inspection for ready detection of initial symptom (initial dementia) of senile dementia.

### BRIEF DESCRIPTION OF THE DRAWINGS

Fig. 1 is a diagram showing one example of a dementia degree examining chart used for a kanji color discerning test;
Fig. 2 is a diagram showing one example of a dementia degree examining chart used for a kana reading test;
Fig. 3 is a perspective view of an outer appearance of a computer system operated as a dementia inspection apparatus of an embodiment of the invention;
Fig. 4 is a block diagram of hardware of a computer system 100 shown in Fig. 3;
Fig. 5 is a functional block diagram of an embodiment of the dementia inspection apparatus realized when a dementia inspection program in the computer system shown in Figs. 3 and 4 is executed;
Fig. 6 is a flowchart showing operation of the dementia inspection apparatus in the inspection of a dementia degree of a subject;
Fig. 7 is a flowchart showing pre-test processing;
Fig. 8 is a diagram showing one example of a first examination chart;
Fig. 9 is a diagram showing one example of a color information table;
Fig. 10 is a diagram showing an example of color code in the color information table;
Fig. 11 is a diagram showing one example of color coded inspection sentences in which sentences are divided into character string by punctuation marks, and the character strings have different colors;
Fig. 12 is a diagram showing one example of a second examination chart which is produced by a second examination chart producing section shown in Fig. 5 and which is shown on a chart display section after the first examination chart shown in Fig. 8;
Fig. 13-A is a diagram showing a first unit group of three kinds of unit groups;
Fig. 13-B is a diagram showing a second unit group of the three kinds of unit groups;
Fig. 13-C is a diagram showing a third unit group of the three kinds of unit groups;
Fig. 14 is a flowchart showing determining processing;
Fig. 15 is a diagram showing a reference value table indicating reference value with respect to each judgment item which is referred to in the determining processing shown in Fig. 14;
Fig. 16 is a diagram showing one example of a pre-test first examination chart and a pre-test second examination chart used in a pre-test;
Fig. 17 is a block diagram of a server/client system operated as one embodiment of a dementia inspection system; and
Fig. 18 is a functional block diagram of an embodiment of the dementia inspection system realized by the server/client system shown in Fig. 17.

### BEST MODE FOR CARRYING OUT THE INVENTION

Embodiments of the present invention will be described below.

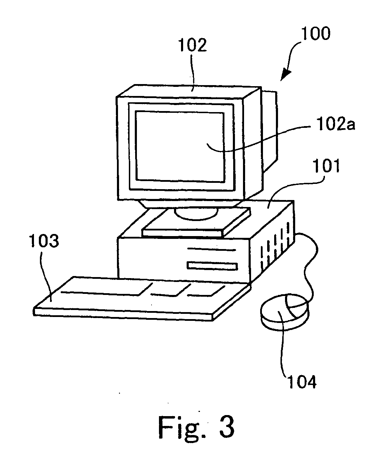

Fig. 3 is a perspective view of an outer appearance of a computer system operated as a dementia inspection apparatus of an embodiment of the invention. The dementia inspection apparatus as one embodiment of the invention is realized by a combination of hardware of a computer system 100 and software executed in the hardware.

The computer system 100 includes a main body 101 in which a CPU, a RAM memory, a magnetic disc, a communication board and the like are incorporated. The computer system 100 also includes a CRT display 102 for displaying information on a display screen 102a upon reception of instructions from the main body, a keyboard 103 through which instructions and character information of a subject or operator are input, and a mouse 104 through which an arbitrary position on the display screen is designated and instructions in accordance with an icon displayed on that position is input.

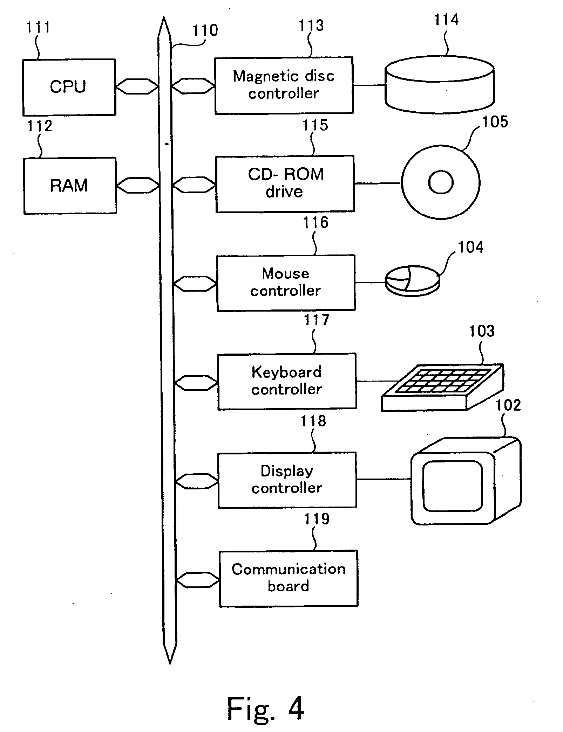

A CD-ROM 105 is removably loaded into the main body 101, and a CD-ROM drive for driving the mounted CD-ROM 105 (see Fig 4) is also incorporated in the main body 101.

A dementia inspection program is stored in the CD-ROM 105. The CD-ROM 105 is loaded into the main body 101, and the dementia inspection program stored in the CD-ROM 105 is installed into a magnetic disc of the computer system 100 by the CD-ROM 105 drive. If the dementia inspection program installed in the magnetic disc of the computer system 100 is started, the computer system 100 is operated as one embodiment of the dementia inspection apparatus of the invention.

Fig. 4 is a block diagram of hardware of the computer system 100 shown in Fig. 3.

In the hardware block diagram, a CPU 111, a RAM 112, a magnetic disc controller 113, a CD-ROM drive 115, a mouse controller 116, a keyboard controller 117, a display controller 118 and a communication board 119, and they are connected to each other through a bus 110.

As explained with reference to Fig. 3, the CD-ROM 105 is loaded into the CD-ROM drive 115, and the CD-ROM drive 115 is used for accessing the loaded CD-ROM 105.

The communication board 119 is connected to a line of communication. A result of inspection of a subject carried out using this computer system is sent to a data storage server (not shown) through the communication board 119, and the result is used for progress observation of subjects and for statistical processing of a large number of subjects.

Fig. 4 also shows a magnetic disc 114 accessed by the magnetic disc controller 113, the mouse 104 controlled by the mouse controller 116, the keyboard 103 controlled by the keyboard controller 117, and the CRT display 102 controlled by the display controller 118.

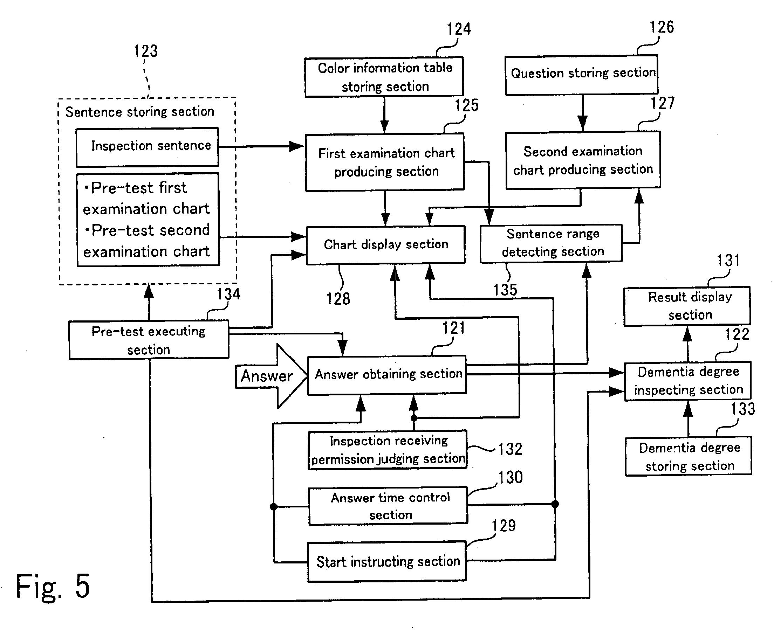

Fig. 5 is a functional block diagram of an embodiment of the dementia inspection apparatus realized when a dementia inspection program in the computer system shown in Figs. 3 and 4 is executed.

An answer obtaining section 121 constitutes the dementia inspection apparatus 120 shown in Fig. 5. The answer obtaining section 121 obtains answers from a subject with respect to both the first examination chart and second examination chart. In terms of hardware, the keyboard 103, the keyboard controller 117, the mouse 104 and the mouse controller 116 shown in Figs. 3 and 4 correspond to the answer obtaining section 121. Here, the first examination chart includes colored inspection sentences which are color coded so that character groups constituting a story having color words representing colors have the same color in each color words. A subject is required to judge whether color of characters constituting the color word is the same as color represented by that color word, and is required to answer in a style in which correction and error can be judged objectively. The second examination chart comprises plural questions concerning the contents of the inspection sentence and plural answers to be selected from answers prepared for each question.

A dementia degree inspecting section 122 examines a dementia degree representing a degree of dementia of a subject based on the answer obtained in the answer obtaining section 121. In terms of hardware, the magnetic disc 114 in which a program for carrying out such examination is stored, and the CPU 111 for executing such examination program correspond to the dementia degree inspecting section 122.

A sentence storing section 123 stores the inspection sentence while removing color, and stores later-described pre-test first examination chart and pre-test second examination chart. In terms of hardware, the magnetic disc 114 shown in Fig. 4 corresponds to the sentence storing section 123.

A color information table storing section 124 stores plural color information tables in which when a character group constituting inspection sentence is divided into plural color sections and colored at the time of generation of the first examination chart, each color section and color of that color section are associated with each other. In the plural color information tables, the manner for color-coding the color sections is the same. However, colors of the color sections corresponding to the color information tables are different. A correct answer table in which correct answers to questions of judgments in all of the inspection sentence as to whether a color corresponding to a color word included in an inspection sentence is the same color represented by that color word are added to each color information table. The color information table storing section 124 manages the plural color information tables by means of serial numbers. In terms of hardware, the magnetic disc 114 shown in Fig. 4 corresponds to the color information table storing section 124.

A first examination chart producing section 125 reads out an inspection sentence from which color is removed from the sentence storing section 123, and selects and reads one of the color information table from the plural color information tables stored in the color information table storing section 124. When the first examination chart producing section 125 selects one of the color information tables, the first examination chart producing section 125 selects a color information table associated with a next number of a number associated with a color information table which was selected when the current subject received last time. In accordance with the selected color information table, the character group constituting the inspection sentence from which color is removed is color coded with plural colors, thereby completing the inspection sentence. The first examination chart producing section 125 adds predetermined question sentence to the inspection sentence, and produces a first examination chart. The first examination chart producing section 125 temporarily stores, in the RAM or the like shown in Fig. 5, a correct answer table added to the color information table used for producing the first examination chart. In terms of hardware, the magnetic disc 114 in which a program for carrying out the processing is stored, and the CPU 111 for executing the processing correspond to the first examination chart producing section 125.

A portion constituted by the sentence storing section 123, the color information table storing section 124 and the first examination chart producing section 125 corresponds to one example of the first examination chart forming section of the invention.

A sentence range detecting section 135 detects a range of the inspection sentence which a subj ect was able to read within a predetermined answer time limit with respect to the first examination chart. In this embodiment, a subject answers by clicking a character in the inspection sentence displayed on a later-described chart display section 128 using the mouse 104. The sentence range detecting section 135 receives an answer from the subject through the answer obtaining section 121, refers to the answer, obtains a character in the inspection sentence clicked by the subj ect within the predetermined answer time limit, refers to the inspection sentence received from the first examination chart producing section 125, and obtains a position of a character closest to an end of the inspection sentence in the clicked characters. A range from a first line in the inspection sentence to a line where this character is described is defined as a sentence range where the subject was able to read within the answer time limit. In terms of hardware, the magnetic disc 114 which stores a program for executing such processing, and the CPU 111 which executes the program for executing the processing correspond to the sentence range detecting section 135.

A question storing section 126 stores plural kinds of question units each comprising a combination of questions concerning a story in the inspection sentences and plural answers selected from prepared answers for the questions. In this embodiment, each question unit is associated with a line in the inspection sentence in which contents concerning the question unit are described. In the embodiment, the plural kinds of question units are divided into plural unit groups comprising a predetermined number of (e.g., five) question units and stored. Further, a correct answer table in which correct answers for the plural question units constituting a unit group are collected is added to each unit group. The question storing section 126 manages the plural unit groups by means of serial numbers. In terms of hardware, the magnetic disc 114 shown in Fig. 4 corresponds to the question storing section 126.

A second examination chart producing section 127 selects one of the plural unit groups stored in the question storing section 126. In selecting one of the unit groups, a second examination chart producing section 125 selects a unit group associated with a next number of a number associated with a unit group which was selected when the current subject received inspection last time. The second examination chart producing section 127 further selects, from the plural question units constituting a unit group which was selected here, a question unit in which a line is associated with a line in a sentence range detected by the sentence range detecting section 135, thereby completing the second examination chart. Of the correct answers described in the correct answer table added to the selected unit group, the second examination chart producing section 127 collects correct answers corresponding to the question units selected as the second examination chart, and completes the correct answer table with respect to the second examination chart. The completed correct answer table is temporarily stored in the RAM shown in Fig. 4 or the like. In terms of hardware, the magnetic disc 114 which stors a program for executing such processing and the CPU 111 which executes the program for executing the processing correspond to the second examination chart producing section 127.

Here, a portion constituted by the question storing section 126 and the second examination chart producing section 127 correspond to one example of the second examination chart forming section of the invention.

The chart display section 128 displays the first examination chart and the second examination chart, and in terms of hardware, the CRT display 102 shown in Figs. 3 and 4 corresponds to the chart display section 128. The first examination chart is first displayed. After the acquisition of answer by the answer obtaining section 121 is completed, the second examination chart is displayed. The processing for displaying the examination charts on the chart display section 128 in this order is carried out by the CPU 111 which executes the program for carrying out the processing.

A start instructing section 129 first displays, on the chart display section 128, a message for inquiring of a subject whether input of answer to the first examination chart should be started. If the subject informed that the input of answer should be started by clicking operation of icon using the mouse 104 or by operating the keyboard 103, the message informing the start of answer input and the answer time limit of answer input is displayed on the chart display section 128 and then, the first examination chart produced by the first examination chart producing section 125 is displayed on the chart display section 128. Also when the answer input to the second examination chart is started, a message for inquiring whether the answer input should be started, a message informing that the answer input is started in accordance with a subj ect' s operation to the message, and a message for informing the answer time limit of the answer input are displayed, and a series of processing for displaying the second examination chart is carried out. In terms of hardware, the magnetic disc 114 which stores a program for executing such processing and the CPU 111 which executes the program for executing the processing correspond to the start instructing section 129.

An answer time control section 130 monitors time elapsed after the first examination chart is displayed on the chart display section 128, and if the elapsed time reaches the predetermined answer time limit, the answer time control section 130 removes the first examination chart from the chart display section 128, and stops the obtaining operation of answer from the subject in the answer obtaining section 121. Further, the answer time control section 130 monitors time elapsed after the second examination chart is displayed on the chart display section 128, and if the elapsed time reaches the predetermined answer time limit, the answer time control section 130 removes the second examination chart from the chart display section 128, and stops the obtaining operation of answer from the subject in the answer obtaining section 121. In terms of hardware, the magnetic disc 114 which stors a program for executing such processing and the CPU 111 which executes the program for executing the processing correspond to the answer time control section 130.

A result display section 131 displays a result of dementia degree determination obtained by the dementia degree inspecting section 122. In terms of hardware, the CRT display 102 shown in Figs. 3 and 4 corresponds to the result display section 131.

An inspection receiving permission judging section 132 permits or prohibits inspection for an inspection wisher depending upon whether a predetermined period of time is elapsed after the last inspection. In terms of hardware, the magnetic disc 114 which stors a program for executing such processing and the CPU 111 which executes the program for executing the processing correspond to the inspection receiving permission judging section 132.

A dementia degree storing section 133 stores correspondence between answers and dementia degree with respect to both the first examination chart and second examination chart, and in terms of hardware, the magnetic disc 114 shown in Fig. 4 and the like corresponds to the dementia degree storing section 133. The dementia degree inspecting section 122 refers to the dementia degree storing section 133, and examines the dementia degree of a subject from answers with respect to both the first examination chart and second examination chart of the current subject obtained by the answer obtaining section 121.

A pre-test executing section 134 executes the pre-test which is a series of processing, e.g., the pre-test executing section 134 reads out the pre-test first examination chart and the pre-test second examination chart from the sentence storing section 123, displays these charts on the chart display section 128, obtains answers to these charts in the answer obtaining section 121, and examines the dementia degree in the dementia degree inspecting section 122 based on the answers to the charts. In terms of hardware, the magnetic disc 114 which stores a program for executing such processing and the CPU 111 which executes the program for executing the processing correspond to the pre-test executing section 134. Here, the pre-test first examination chart and the pre-test second examination chart respectively have the same structures as the first examination chart and second examination chart. In this regard, the first examination chart and the second examination chart are produced whenever the examination is carried out by the first examination chart producing section 125 and the second examination chart producing section 127, but in the case of the pre-test first examination chart and the pre-test second examination chart, previously formed charts are merely stored in the sentence storing section 123. The correct answer tables in which correct answers to the examination charts are respectively added to the pre-test first examination chart and the pre-test second examination chart.

The dementia inspection apparatus 120 shown in Fig. 5 has the above-described structure, and when the dementia degree of a subject is to be inspected, the dementia inspection apparatus 120 is operated as follows:

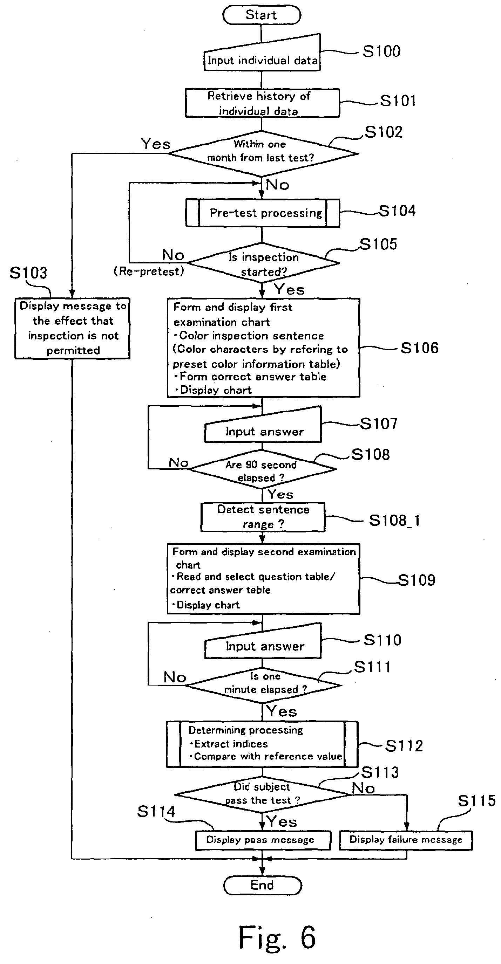

Fig. 6 is a flowchart showing the operation of the dementia inspection apparatus in the inspection of the dementia degree of a subject.

First, an inspection wisher inputs individual data such as name, age, sex, occupation and the like (step S100). When an inspection wisher once receives inspection, he or she is informed of ID number. In this embodiment, a subject can input the above-described personal information, and can also input the ID number instead of the personal information. Therefore, an inspection wisher who has received the inspection before can input the ID number instead of the personal information.

If the personal information or ID number is input, the inspection receiving permission judging section 132 retrieves past inspection history concerning that inspection wisher (step S101). Next, it is determined whether the inspection wisher received inspection during a predetermined period, e.g., within last one month (step S102) . When the inspection wisher received inspection within one month (Yes in step S102), the inspection receiving permission judging section 132 displays a message to the effect that the inspection wisher cannot receive inspection (step S103) on the chart display section 128, and the inspection processing of the current dementia degree is completed. This is because that if a subject received plural inspections within a short period of time, there is no effect, and if the subject becomes used to the inspection, there is an adverse possibility that erroneous inspection result may come out.

When the inspection wisher receives inspection for the first time, or one month is elapsed after the last inspection (No in step S102), inspection is permitted.

If the inspection is permitted and the inspection wisher becomes a subject, the pre-test first examination chart and the pre-test second examination chart are carried out for the subj ect before the real test using the first examination chart and the second examination chart (step S104). Details of the pre-test will be explained later.

When the pre-test is completed, the start instructing section 129 requires the subject to determine whether the real test should be started or the pre-test should be carried out again (step S105). If the subject requests the pre-test again (No in step S105), pre-test is again carried out (step S104), and if this pre-test is completed, the subject is again required to determine whether the real test should be started. As long as the subject requests the pre-test, the pre-test is repeated over and over again. With this, the subject can learn the operation of the dementia inspection apparatus.

In step S105, if the subject requests the start of the real test (Yes in step S105), a first examination chart is produced in the first examination chart producing section 125 and the first examination chart is displayed on the chart display section 128 (step S106). As explained with reference to Fig. 5, inspection sentence which is read out from the sentence storing section 123 and from which color is removed is colored in accordance with a color information table which is read out from the color information table storing section 124, and the inspection sentence is completed. A predetermined question sentence is added to this inspection sentence, and the first examination chart is formed. As explained with reference to Fig. 5, the color information table used here is temporarily stored in the RAM 112 shown in Fig. 4 as a correct answer table with respect to the first examination chart. Details of the first examination chart and the correct answer table will be explained later with reference to an actual example. A message informing the subject that input of answers to the first examination chart is started and a message informing the subject of the answer time limit (90 seconds) of input of answers are displayed on the chart display section 128 by the start instructing section 129 and then, the first examination chart is displayed on the chart display section 128. As explained with reference to Fig. 5, the first examination chart producing section 125 selects one of the color information tables which is different from that used in the last inspection, and the first examination chart is formed in accordance with the color information table for the same subject. This is because that if a test is carried out every time while using inspection sentence colored with the same pattern, the subject learns answers to the first examination chart, and there is an adverse possibility that erroneous inspection result may come out.

If the first examination chart is displayed on the chart display section 128, the subject operates the keyboard 103 or the mouse 104 constituting the answer obtaining section 121, and inputs answers to the first dementia degree examining chart (step S107). If the first examination chart is displayed on the chart display section 128, the answer time control section 130 starts monitoring the elapsed time. The answer time control section 130 determines whether the elapsed time reaches 90 seconds (the answer time limit of the answer input) (step S107). When the elapsed time has not yet reached 90 seconds (No in step S108), the first examination chart is left displayed on the chart display section 128, and obtaining operation of answer from the subject is continued. If the elapsed time reaches 90 seconds (Yes in step S108), the first examination chart is removed from the chart display section 128, and the obtaining operation of answer in the answer obtaining section is stopped. Then, the answer obtaining section 121 transfers the answers input within the answer time limit to the dementia degree inspecting section 122 and the sentence range detecting section 135.

Next, the sentence range detecting section 135 detects a range in the inspection sentence which the subject was able to read within the predetermined answer time limit (90 seconds) to the first examination chart (step S108_{_}1). The sentence range detecting section 135 refers to the answers from the subject received from the answer obtaining section 121, and obtains a position of a character which is closest to the sentence end in plural characters in the inspection sentence which the subj ect clicked within the predetermined answer time limit. A range from the first line in the inspection sentence to a line where this character exists is defined as a sentence range which the subject was able to read within the answer time limit. The sentence range detecting section 135 transfers information representing the sentence range to the second examination chart producing section 125.

Next, the second examination chart producing section 125 produces a second examination chart and the second examination chart is displayed on the chart display section 128 (step S109). As explained with reference to Fig. 5, each question unit is composed of questions concerning a story in inspection sentence and plural answers prepared to the questions. The question unit is divided into plural unit groups, and each unit group has a predetermined number of question units. The question storing section 126 stores therein the unit groups. The second examination chart producing section 125 reads out one of the unit groups from the question storing section 126. From the plural question units constituting the selected unit group, the second examination chart producing section 125 further selects a question unit which corresponds to a line in the sentence range detected by the sentence range detecting section 135, thereby completing the second examination chart. As explained with reference to Fig. 5, of correct answers described in the correct answer table added to the selected unit group, the second examination chart producing section 125 collects correct answers corresponding to each question unit selected as the second examination chart, and a correct answer table with respect to the second examination chart is completed. The completed correct answer table is temporarily stored in the RAM shown in Fig. 4 or the like, a message informing the subject that input of answers to the second examination chart is to be started and a message informing the subject of the answer time limit (one minute) for inputting answers are displayed on the chart display section 128 by the start instructing section 129 and then, the second examination chart is displayed on the chart display section 128. Details of the second examination chart and the correct answer table will also be explained later with reference to actual example. Here, as explained with reference to Fig. 5, the second examination chart producing section selects one of unit groups which is different from that used when the subject received inspection last time, and using the selected unit group, the current second examination chart is formed for the same subject. This is because that if a test is carried out every time while using the same second examination chart, the subject learns answers to the second examination chart, and there is an adverse possibility that erroneous inspection result may come out.

When the second examination chart is displayed on the chart display section 128, then, the subject inputs an answer (step S110). As described above, answer time limit is set for the answer to the second examination chart. In this embodiment, the answer time limit for the second examination chart is one minute. The answer time control section 130 determines whether time elapsed after the second examination chart is displayed on the chart display section 128 reaches one minute (answer time limit) (step S111), and if the elapsed time is less than one minute (No in step S111), the answer obtaining section allows the subject to input answer, and if the elapsed time reaches one minute (Yes in step S111), the second examination chart is removed from the chart display section 128, and the answer obtaining operation of the answer obtaining section is stopped. The answer obtaining section 121 transfers the answer which is input within the answer time limit to the dementia degree inspecting section 122.

If the answers to both the first examination chart and second examination chart are completed in this manner, the dementia degree inspecting section 122 determines the dementia degree of the subject (step S112). In this determining processing, correct answer tables of the first examination chart and second examination chart which are temporarily stored in the RAM 112 shown in Fig. 4 are read out, the correct answer tables and answers from the subject are compared with each other, thereby extracting plural kinds of indices which are reasons of determination of the dementia degree, and based on the indices and the comparison of reference values with respect to the indices, it is determined whether the subject passes or fails the test. In this embodiment, the reference values with respect to the respective indices are stored in the dementia degree storing section 133 shown in Fig. 5. In the above determination, "pass" means that the subj ect is not suspected of dementia, and "failure" means that the subject is suspected of dementia and workup is necessary. Details of the determining processing will also be explained later.

In accordance with pass or failure of the judging result of the processing in step S112 (S113), if the result is pass (Yes in step S113), a message to that effect is displayed on the result display section 131 (step S114), and the dementia degree determining processing is completed. If the result is failure (No in step S113), a message to that effect and a message to the effect that workup is necessary are displayed on the result display section 131 (step S15), the dementia degree determining processing is completed.

Next, details of the pre-test processing shown in Fig. 6 will be explained.

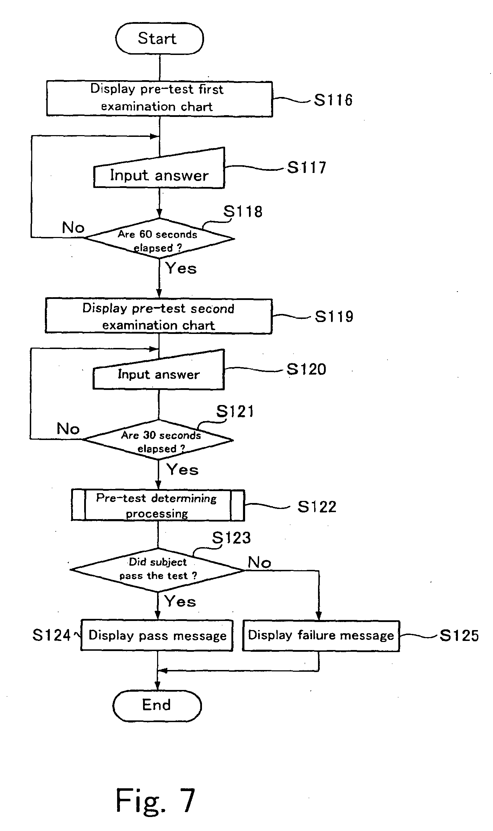

Fig. 7 is a flowchart showing the pre-test processing.

If the pre-test processing is started, the pre-test executing section 134 first reads out the pre-test first examination chart from the sentence storing section 123, and displays the same on the chart display section 128 (step S116). If the pre-test first examination chart is displayed, the obtaining operation of answer from the subject is started by the answer obtaining section (step S117), and monitoring of time elapsed after the pre-test first examination chart is displayed is started (step S118). If the elapsed time is less than 60 seconds (No in step S118), the obtaining operation of answer is continued, and if the elapsed time reaches 60 seconds (Yes in step S118), the pre-test first examination chart is removed from the chart display section 128, and the obtaining operation of answer in the answer obtaining section is stopped. Answers from the subject received by the answer obtaining section within 60 seconds are transferred to the dementia degree inspecting section 122.

Next, the pre-test executing section 134 reads out the pre-test second examination chart from the sentence storing section 123, and displays the same on the chart display section 128 (step S119). The answering processing (step S120) by the subject to the pre-test second examination chart and monitoring processing (step S121) of time elapsed after the pre-test second examination chart is displayed are the same as those in step S117 and step S118 and thus, explanation thereof will be omitted. The answer time limit in the processing in step S121 is 30 seconds.

Based on answers to the pre-test first examination chart and the pre-test second examination chart made by the subject within the answer time limits, the dementia degree inspecting section 122 determines whether the subject passes or fails the test (step S122). This determining processing is the same as the determining processing (step S112) whether pass or fail in the real test explained with reference to Fig. 6 and thus, explanation thereof will be omitted. In this regard, the correct answer tables for the pre-test first examination chart and the pre-test second examination chart used in this determining processing are added to the pre-test first examination chart and the pre-test second examination chart, respectively as explained with reference to Fig. 5.

The message displaying processing (step S123) based on the inspection result, the displaying processing of pass message (step S124), the displaying processing of failure message (S 125) after step S122 are the same as those in step S113, step S114 and step S115 shown in Fig. 6, respectively and thus, explanation thereof will be omitted.

Next, details of the first examination chart shown in Fig. 6 will be explained.

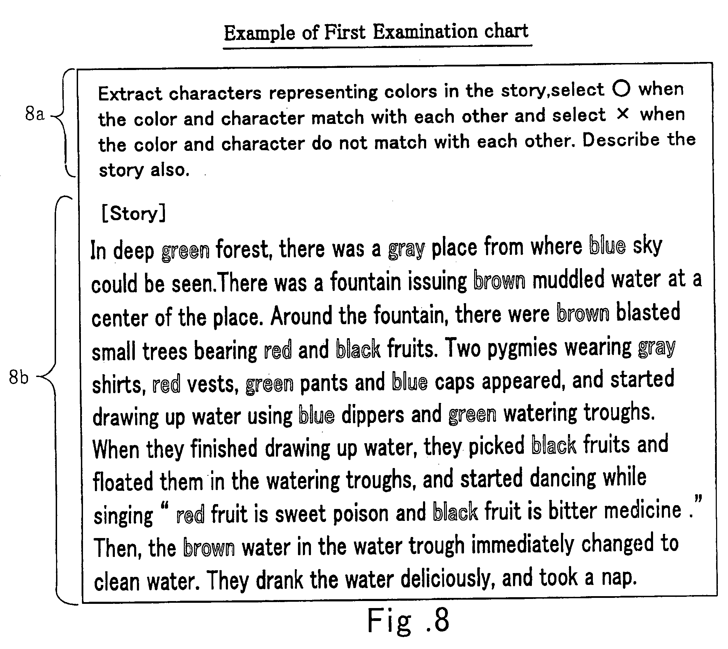

Fig. 8 is a diagram showing one example of the first examination chart which is produced by the first examination chart producing section shown in Fig. 5 and displayed on the chart display section.

The first examination chart shown in Fig. 8 comprises an inspection sentence 8b and a question sentence 8a. The inspection sentence 8b includes character groups constituting a story including color words representing colors. In the character group, color words are colored in different colors, and characters other than color words are colored in black. In the question sentence 8a, a subject is required to determine whether color of a color word is the same color represented by this color word, and the subject is required to answer in a style in which correct or error of the determination can be found objectively.

In the plural color words included in the inspection sentence 8b, colors represented by the color words may match with colors of the color words themselves in some cases, and may mismatch in other cases. For example, some of the characters "green" are colored in green, and other characters "green" are colored in different colors other than green. The same is applied to other color words "red", "brown", "blue" and the like.

In accordance with instructions of the question sentence 8a, the subject first extracts color words from the inspection sentence 8b, checks whether color represented by that color word matches with color of that color word one by one, and clicks that color word using the mouse 104 shown in Fig. 4, thereby inputting result. That is, the subject is required to exhibit three kinds of attention distribution abilities, i.e., the subject is required to extract a color word, to understand the meaning of the extracted color word, and to determine color of the color word. Such an inspection is effective for determining the function of a frontal lobe causing dementia.

Here, the sentence range detecting section 135 shown in Fig. 5 extracts plural color words in an inspection sentence clicked by the subj ect within the predetermined answer time limit, or extracts a character closest to a sentence end of an inspection sentence among characters which are erroneously clicked. Then, a range from the first line in the inspection sentence 8b to a line where this character exists is defined as a sentence range that the subject was able to read within the answer time limit.

In this embodiment, colors of the color words are set different for each test. With this, repetition learning effect of a subject can be suppressed.

As instructed in the question sentence 8a, a subject is also required to exhibit another attention distributing ability, i.e., the subject is required tomemorize a story in the inspection sentence 8b.

Of these attention distributing abilities, two attention distributing abilities except the memorizing of a story are evaluated from answers from the subject made during the predetermined answer time limit (90 seconds in this embodiment).

The memorizing ability of a story is determined by the later-described second examination chart.

Next, details of the color information table which is referred to when the first examination chart producing section 125 shown in Fig. 5 produces the first examination chart will be explained.

As described above, the color information table storing section 124 stores the plural color information tables. In accordance with one of the color information tables which is read from the color information table storing section 124, inspection sentence from which color is removed is colored, thereby completing the inspection sentence. By adding a predetermined question sentence to this inspection sentence, the first examination chart is produced. In the following explanation, an example of the color information table will be explained.

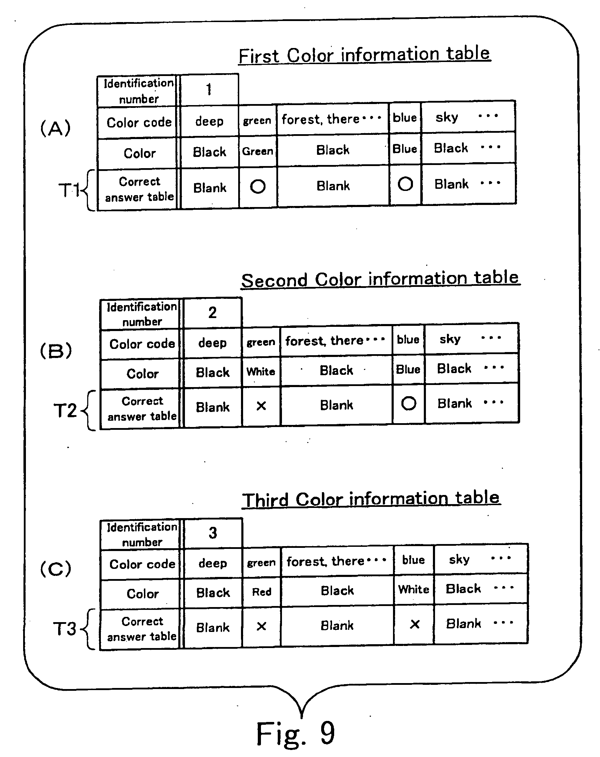

Fig. 9 is a diagram showing an example of the color information table.

In parts (A) to (C) of Fig. 9, first to third color information tables are shown. In each of the color information tables shown in Fig. 9, plural colors are associated with color sections which are color words representing colors in the common inspection sentence from which colors are removed, and color sections which are characters other than the color words are all associated with a single color "black".

In this example, the color word corresponds to one example of a color word of the invention.

Correct answer tables T1 to T3 in which correct answers in the first examination chart produced using the color information tables are added to the color information tables, respectively. Here, the correct answers shown in the color information table shown in Fig. 9 is for the question sentence 8a in Fig. 8.

In the first color information table shown in part (A) of Fig. 9, color words representing "green" and "blue" shown in the table are associated with green and blue, respectively. In the correct answer table T1 added to the first color information table, characters other than the color words are associated with "blanks", color words representing "green" and "blue" are both associated with "○" (true) because colors of the color words match with colors represented by the color words, respectively. Further, 1 of serial numbers 1 to 3 is associated to the first color information table as identification number.

In the second color information table shown in part (B) of Fig. 9, color words representing "green" and "blue" shown in the table are associated with white and blue, respectively. In the correct answer table T2 added to the second color information table, characters other than the color words are associated with "blanks", a color word representing "green" is associated with "x" (false) because color of the color word and color represented by this color word do not match with each other. A color word representing "blue" is associated with "○" because color of this color word matches with color represented by this color word. Further, 2 is associated to the second color information table as identification number.

In the second color information table shown in part (C) of Fig. 9, color words representing "green" and "blue" shown in the table are associated with colors which are different from those represented by the respective color words, and in the added correct answer table T3, these color words are associated with "×". Characters other than color words are associated with "blanks". The third color information table is associated with 3 as identification number.

In this embodiment, the three kinds of color information tables are stored in the color information table storing section 124 shown in Fig. 5. The first examination chart producing section 125 selects one of the three kinds of color information tables, and the inspection sentence from which color is removed is tinted with color in accordance with the color information table. When the color information table is selected, a color information table associated with a next number of a number associated with a color information table selected by the current subject when the subject received the inspection last time. For example, if the number associated with a color information table selected in received last time is 1, a color information table selected this time is a second color information table associated with 2. With this, erroneous diagnosis caused if a subject becomes used to the test is suppressed.

In the explanation of this embodiment, as the color codes in the plural color information tables, color words representing color are associated with plural colors, and characters other than color words are associated with a single color. However, the present invention is not limited to this color code. Color-codes other than the color code explained in the embodiment will be explained briefly.

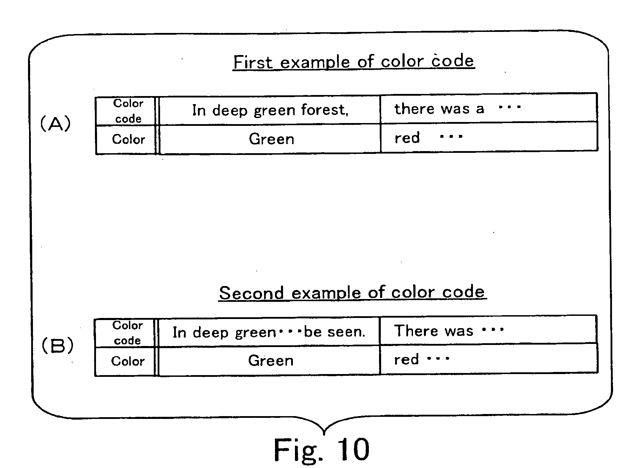

Fig. 10 is a diagram showing color code in the color information table.

Fig. 10 shows two kinds of color codes.

Part (A) of Fig. 10 shows an example of color code in which inspection sentence from which color is removed is colored such that character strings divided by commas in the sentence are associated with different colors. In Part (A) of Fig. 10, a character string from the top of the sentence to a comma is associated with green including this comma, and characters after this comma are associated with red.

Part (B) of Fig. 10 shows an example of color code in which inspection sentence from which color is removed is colored such that character strings divided by periods in the sentence are associated with different colors. In Part (B) of Fig. 10, a character string from the top of the sentence to a period is associated with green including this period, and characters after this period are associated with red.

The commas and periods in the examples of color codes shown in parts (A) and (B) of Fig. 10 correspond to one example of specific kinds of characters of the present invention.

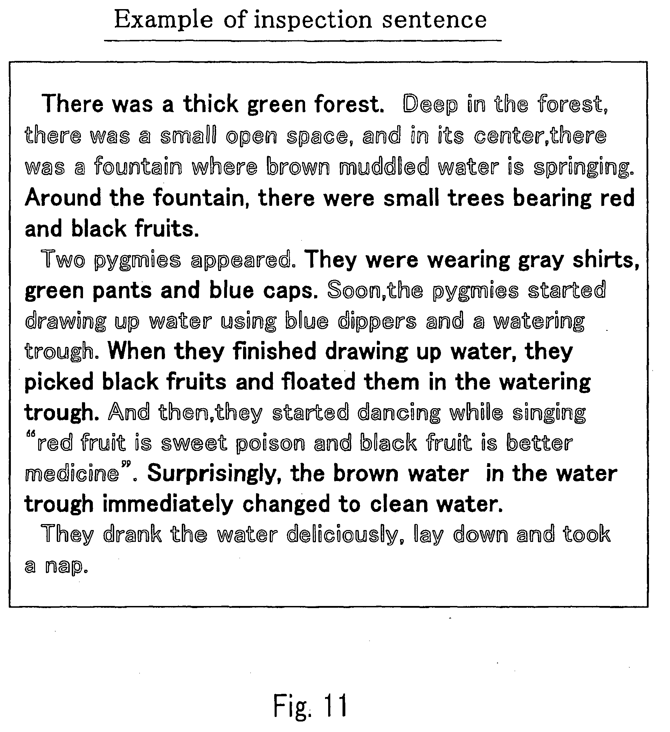

Fig. 11 shows one example of the inspection sentence in which character strings divided by the periods in the sentence are tinted with different colors. This corresponds to the example of color code shown in part (B) of Fig. 10. The inspection sentence shown in Fig. 11 and the inspection sentence shown in Figs. 8 and 9 are slightly different from each other in stories represented by respective sentences. However, this is not related to the essence of the present invention.

It is known that recognition of color represented by a color word in an inspection sentence colored in this manner strongly receives interference of color of character around this color word, and it is more difficult to swiftly determine whether a color represented by the color word and color of that color word itself match or mismatch with each other as compared with a case in which only color words are colored. That is, if the inspection sentence shown in Fig. 8 is used, it is possible to carry out a test having higher difficulty level.

The concept of the first and second examination charts of the present invention explained above can also be applied to other language such as English.

Next, details of the second examination chart will be explained.

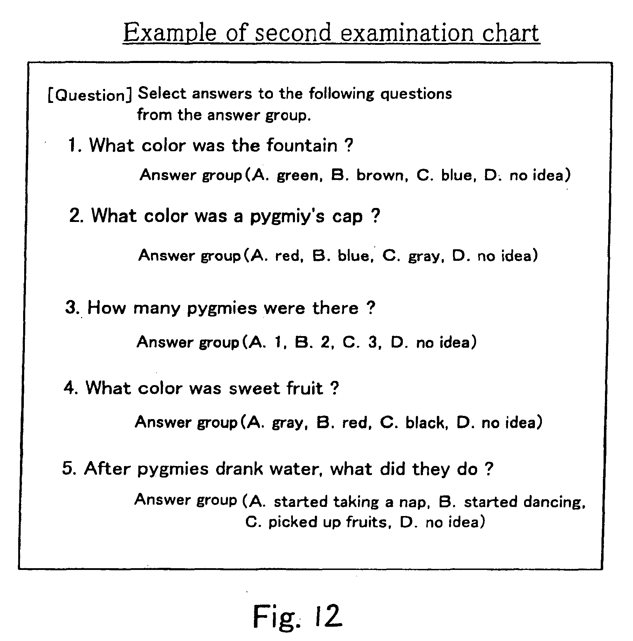

Fig. 12 is a diagram showing one example of the second examination chart which is produced by the second examination chart producing section shown in Fig. 5, and which is displayed on the chart display section after the first examination chart shown in Fig. 8.

Fig. 12 shows the second examination chart comprising five questions and answer groups each having plural answers to be selected for respective questions.

The attention distributing ability, i.e., memorizing ability of a story in the inspection sentence 8b shown in Fig. 8 is determined from answers made by a subject within a predetermined answer time limit (one minute in this embodiment) with respect to the second examination chart.

In the second examination chart shown in Fig. 12, each answer group prepared with each question is provided with an alternative "no idea" for representing that a subject does not know. This suppresses a case in which a subj ect selects a correct answer by coincidence although the subject does not know the answer, and it is possible to prevent the reliability of inspection result of the dementia degree frombeing deteriorated.

Next, the unit group which is referred to when the second examination chart producing section 127 shown in Fig. 5 produces the second examination chart will be explained.

As explained with reference to Figs. 5 and 6, a question storing section 126 stores plural question units each comprising a combination of questions concerning a story in the inspection sentences and plural answers prepared for the questions. The plural kinds of question units are divided into plural unit groups comprising a predetermined number of question units and stored in the question storing section 126. The second examination chart producing section 127 selects one of the unit groups from the question storing section 126, and produces the second examination chart based on the unit group. In this embodiment, later-described three kinds of unit groups are stored in the question storing section 126. When the second examination chart producing section shown in Fig. 5 produces the second examination chart, the three unit groups are referred to. Examples of the three unit groups will be explained below.

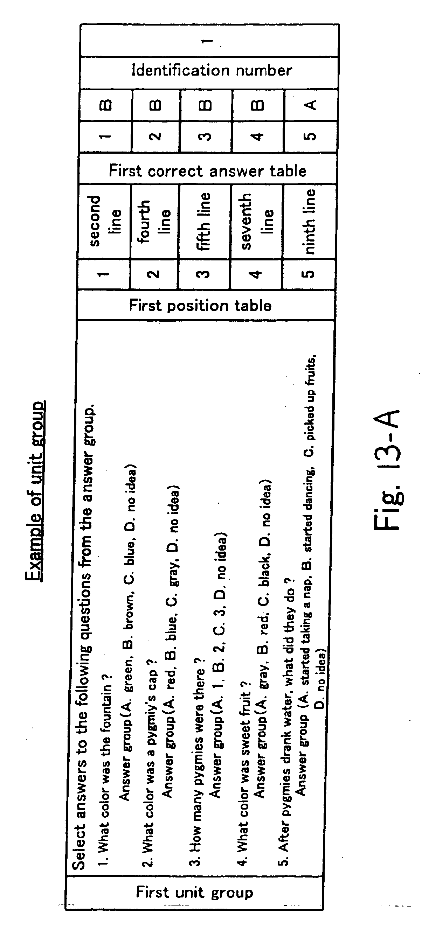

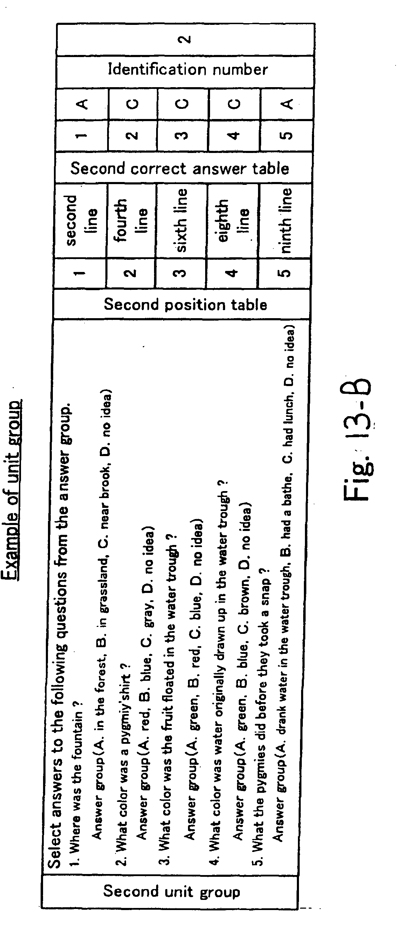

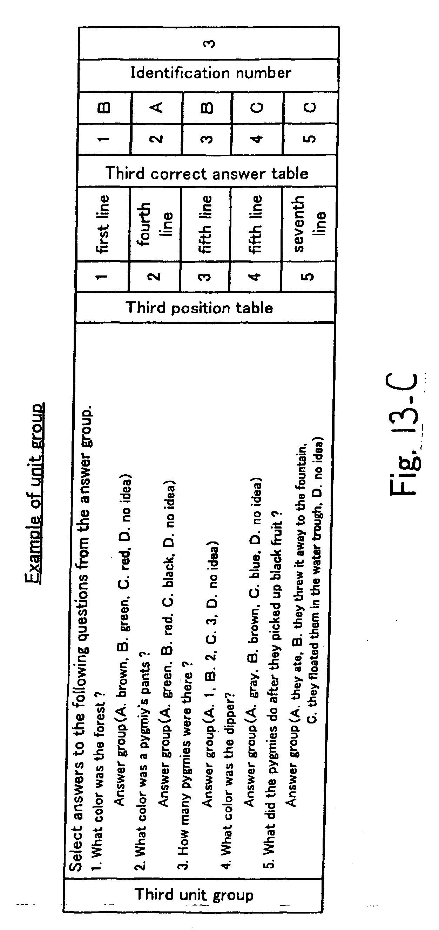

Fig. 13-A shows a first unit group of the three kinds of unit groups. Fig. 13-B shows a second unit group of the three kinds of unit groups. Fig. 13-C shows a third unit group of the three kinds of unit groups.

The question units constituting the three kinds of unit groups shown in Fig. 13-A to Fig. 13-C are different from each other. Each unit group comprises five question units. Each question unit is associated with line in which contents in the inspection sentence 8b shown in Fig. 8 concerning each question unit is described. For example, a correspondence relation between each question unit constituting the first unit group shown in Fig. 13-A and a line in the inspection sentence 8b is as follows. That is, a question unit in a first item is associated with a second line of the inspection sentence 8b shown in Fig. 8, a question unit in a second item is associated with a fourth line, a question unit in a third item is associated with a fifth line, a question unit in a fourth item is associated with a seventh line, and a question unit in a fifth item is associated with a ninth line. This correspondence relation is described in a first position table, and the first position table is added to this unit group. A second position table and a third position table in which the question units constituting the respective unit groups and the contents concerning the question units described in the inspection sentence 8b are added to the unit groups shown in Fig. 13-B and Fig. 13-C, respectively.

Correct answer tables corresponding to respective unit groups are added to the respective unit groups. Each unit group is associated with one of serial numbers 1 to 3 as an identification number.

In this embodiment, the three kinds of unit groups are stored in the question storing section 126 shown in Fig. 5. The second examination chart producing section 127 first selects one of the three kinds of unit groups. In this embodiment, in the sentence range detecting section 135, a sentence range in the inspection sentence which a subject was able to read within a predetermined answer time limit (90 seconds) with respect to the first examination chart is detected. Here, this sentence range is expressed as a line number from a first line in the inspection sentence.

The second examination chart producing section 127 further selects, from plural question units constituting the selected unit group, a question unit in the sentence range detected by the sentence range detecting section 135 where a line indicated by the position table exists, thereby completing the second examination chart. For example, suppose that the first unit group shown in Fig. 13-A is selected by the second examination chart producing section 127. And suppose that the sentence range detected by the sentence range detecting section 135 exists from first to third lines. In this case, the second examination chart producing section 127 further selects a question unit existing from the first to third items in which the line indicated by the first position table exists in the sentence range from the plural question units constituting the unit group, thereby completing the second examination chart. This suppresses a case in which a subject selects a correct answer by coincidence although the subject was unable to read that portion, and it is possible to prevent the reliability of inspection result of the dementia degree from being deteriorated.

The correct answer table with respect to the completed second examination chart is produced as follows. For example, suppose that in the second examination chart completed here, a question unit from the first to third items is selected from the plural question units constituting the first unit group. At this time, correct answers corresponding to the question units from the first to third items are selected from correct answers described in the first correct answer table added to the first unit group, thereby producing the correct answer table with respect to the second examination chart.

Such a second examination chart is displayed on the chart display section 128 shown in Fig. 5. Here, the second examination chart shown in Fig. 12 corresponds to the first unit group shown in Fig. 13-A. In the second examination chart shown in Fig. 12, all of the five question units are shown. This means that the subject read the inspection sentence 8b to the end thereof in the inspection using the first examination chart shown in Fig. 8 before the second examination chart is produced.

When a unit group is to be selected, a unit group associated with a next number of a number associated with a unit group selected when the current subject received inspection last time is selected. For example, when the number associated with a unit group selected last time is 1, a unit group to be selected this time is a second unit group associated with 2. With this, erroneous diagnosis caused when a subject becomes used to the test is suppressed.

With the above procedure, explanation of the first and second examination charts used for inspection of the dementia degree is completed.

Next, details of the determining processing for determining the dementia degree of a subject based on answers of the subject with respect to the first and second examination charts will be explained. This determining processing corresponds to step S112 of the flowchart shown in Fig. 6.

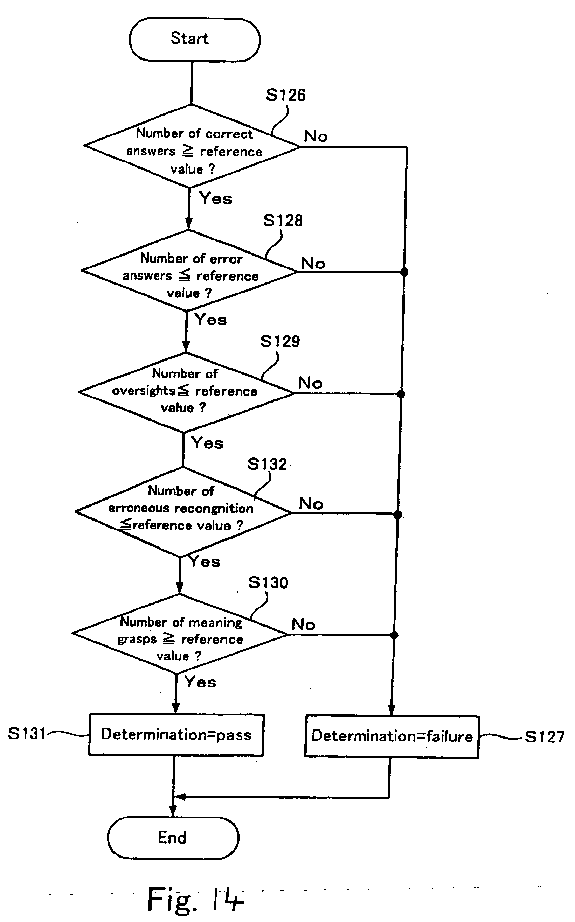

Fig. 14 is a flowchart showing the determining processing.

When the determining processing is started, a later-described plurality of indices are acquired from answers of a subject with respect to the first and second examination charts, and the indices and reference values which are described in the correct answer table stored in the RAM shown in Fig. 4 and which correspond to the indices are compared with each other and determined. First, determining processing based on answers from a subject with respect to the first examination chart is carried out.

First, a subject answers "○" (true) or "x" (false) for color words within a predetermined answer time limit (90 seconds), and of the answers, it is determined whether the number of correct answers which are the number of answers matching with "○" or "×" described in the correct answer table is equal to or higher than the later-described reference value (step S126). If the number of correct answers is less than the reference value (No in step S126), the inspection result is fail (step S127), and the determining processing is completed.

If the number of correct answers is equal to or higher than the reference value (Yes in step S126), a subject answers "○" or "×" for color words within a predetermined answer time limit (90 seconds), and of the answers, it is determined whether the number of error answers which is the number of answers not matching with "○" or "×" described in the correct answer table is equal to or lower than a later-described reference value (step S128). If the number of error answers exceeds the reference value (No in step S128), the determining processing is completed through step S127.

If the number of error answers is equal to or less than the reference value (Yes in step S128), it is determined whether the number of color words which the subject missed from color words included in the inspection sentence, i.e., the number of blanks which were considered to be input by the subject as answers irrespective of color words is equal to or less than the reference value (step S129). Here, the number of color words included in a portion that the subject was not able to read within the answer time limit is also included in the number of oversights. As a result of this determination, if the number of oversights exceeds the reference value (No in step S129), the determining processing is completed through step S127.

If the number of oversights is equal to or less than the reference value (Yes in step S129), it is determined whether the number of characters which erroneously recognized by the subject, i.e., the number of erroneously recognized characters which is the number of subject's answers of "○" or "×" although characters are other than color words is equal to or less than a later-described reference value (step S132). As a result of the determination, if the number of erroneous recognition exceeds the reference value (No in step S132), the determining processing is completed through step S127.

If the number of erroneous recognition is equal to or less than the reference value (Yes in step S132), determining processing based on answers from the subject with respect to the second examination chart is carried out.

Here, it is determined whether the number of grasps which is the number of correct answers to questions concerning a story included in the inspection sentence is equal to or higher than a later-described reference value (step S130). As a result of the determination, if the number of meaning grasps is less than the reference value (No in step S130), the determining processing is completed through step S127.

If the number of meaning grasps is equal to or higher than the reference value (Yes in step S130), it is determined that the subject passes the test (step S131), and the determining processing is completed.

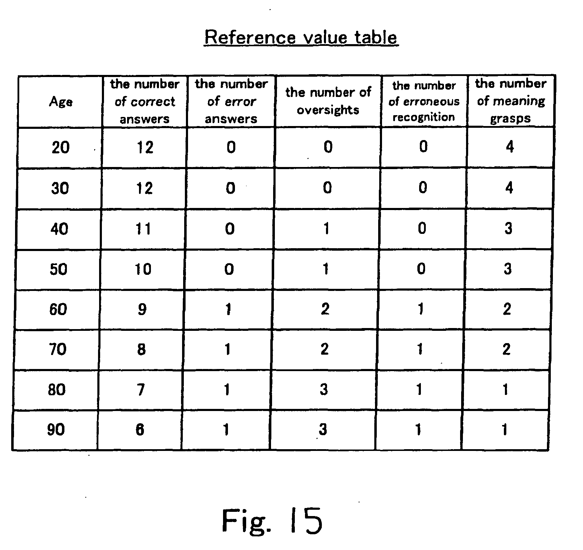

Fig. 15 is a diagram showing a reference value table showing the reference values with respect to various judgment items which are referred to in the determining processing shown in Fig. 14.

In the reference value table shown in Fig. 15, reference values of indices of five items, i.e., the number of correct answers, the number of error answers, the number of oversights, the number of erroneous recognition and the number of meaning grasps are shown organized according to ages from twenties to nineties in increments of ten. In this embodiment, the determining processing is carried out while referring to a reference value suitable for age of a subject.

The reference value table shown in Fig. 15 is stored in the dementia degree storing section 133 shown in Fig. 5 as described above.

The determining processing with respect to the indices of five items, i.e., the number of correct answers, the number of error answers, the number of oversights, the number of erroneous recognition and the number of meaning grasps have been explained with reference to Figs. 14 and 15. Instead of the determining processing with respect to the indices of the five items, it is possible to further extract the number of unclear answers indicating how many alternatives "no idea" a subject selected for plural questions in the second examination chart, and to carry out the later-described determining processing using the number of unclear answers. If such determining processing is carried out, since the precision of the inspection result of the dementia degree is further enhanced, it is preferable to carry out the determining processing using the number of unclear answers.

The examination of the precise dementia degree using the number of unclear answers is carried out by using the number of meaning grasps and the number of unclear answers, and by obtaining a ratio of meaning grasps which is a ratio of correct answers to the total number of answers made by a subject with respect to the second examination chart. That is, using the total number of questions shown to a subject in the second examination chart, the number of unclear answers and the number of meaning grasps are used, and the ratio of meaning grasps is calculated using the following equation:

A ratio of meaning grasps = number of meaning grasps / (total number of questions number of unclear answers)

The ratio of meaning grasps is an index which more precisely indicates how much a subject understands the inspection sentence. In the preferable embodiment, in addition to the comparison determination between the indices of the five items and the reference values shown in Fig. 15, if the ratio of meaning grasps is compared with the reference values (not shown), it is possible to obtain inspection result of more precise dementia degree.

The explanation of the determining processing of dementia degree is completed.

Next, a pre-test examination chart used in the pre-test carried out before the real test will be explained.

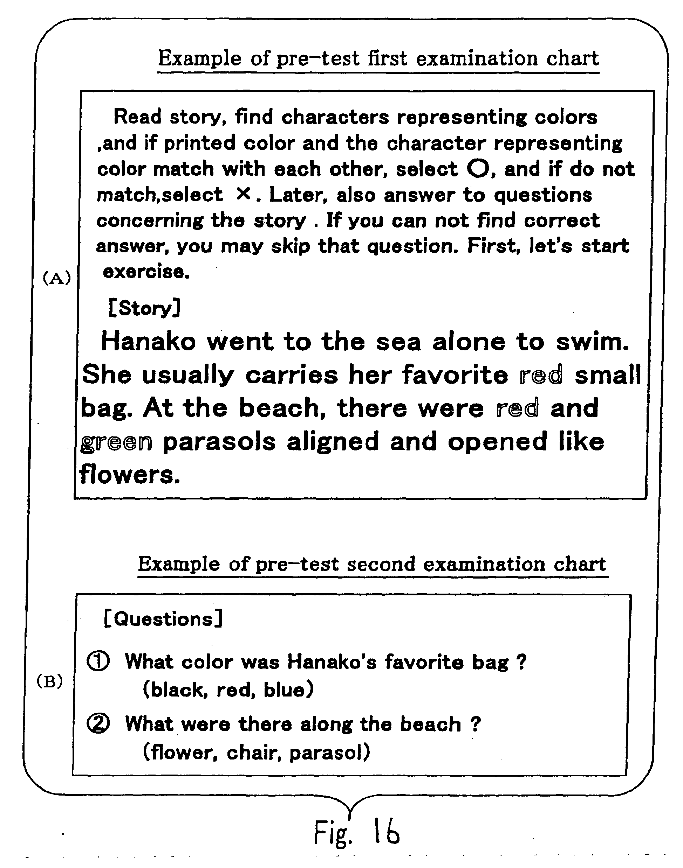

Fig. 16 is a diagram showing one example of the pre-test first examination chart and the pre-test second examination chart used in the pre-test.

Part (A) of Fig. 16 shows the pre-test first examination chart and part (B) of Fig. 16 shows the pre-test second examination chart.

The pre-test first examination chart and the pre-test second examination chart have the same structures as those of the first examination chart and the second examination chart, respectively.

In the embodiment, for the sake of allowing a subject to become used to the operation of the dementia inspection apparatus 100, a pre-test using the pre-test examination chart is carried out before the real test. Since details of the processing have been explained with reference to the flowchart in Fig. 7, explanation thereof will be omitted here.

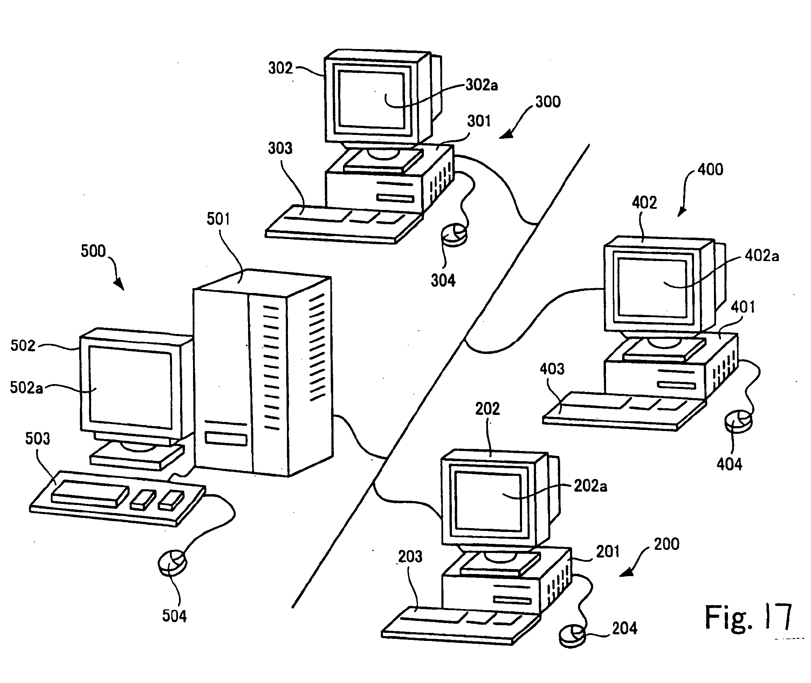

Fig. 17 is a block diagram of the server/client system operated as one embodiment of the dementia inspection system.

Fig. 17 shows three computer systems 200 , 300 , 400 which are operated as dementia inspection clients, and one computer system 500 operated as a dementia inspection server. These computer systems are connected to line of communication.

The computer systems 200, 300, 400, 500 respectively have: main bodies 201, 301, 401, 501 in each of which a CPU, a RAM, a magnetic disc, a communication board and the like are incorporated; CRT displays 202, 302, 402, 502 for displaying screens on display screens 202a, 302a, 402a, 502a in accordance with instructions from the main bodies; keyboards 203, 303, 403, 503 through which instructions of a subject or other operator and character information are input into the computer systems; and mouse 204, 304, 404, 504 for designating an arbitrary position on the display screen to input instructions in accordance with icon or the like displayed on that position.

Since hardware structures of the computer systems 200, 300, 400, 500 are substantially the same as that of the computer system 100 (see Fig. 2), detailed explanation will be omitted here.

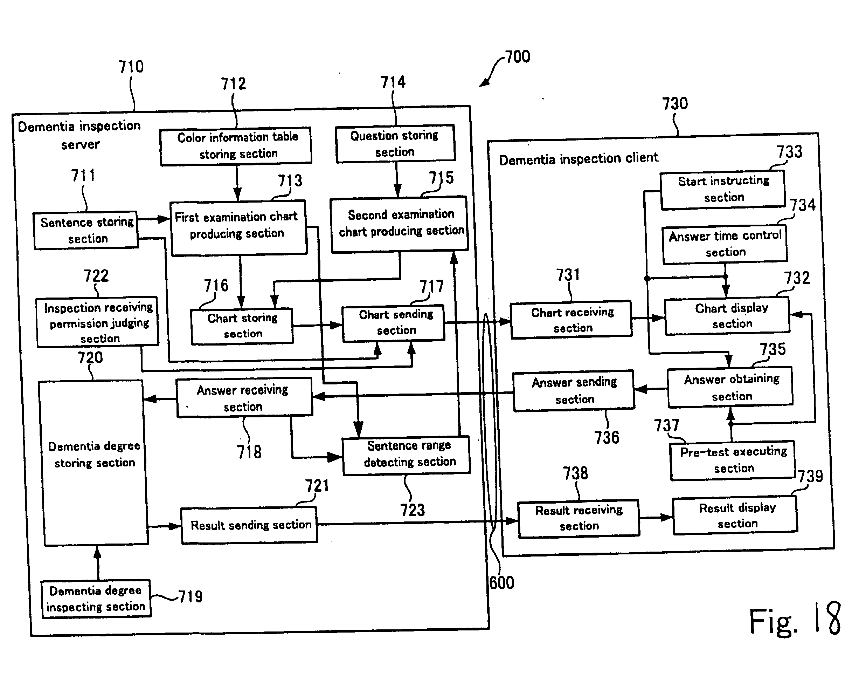

Fig. 18 is a functional block diagram of an embodiment of the dementia inspection system realized by the server/client system shown in Fig. 17. Only one of the dementia inspection clients is shown as a representative.

A dementia inspection system 700 shown in Fig. 18 comprises a dementia inspection server 710 and a dementia inspection client 730.

The dementia inspection server 710 constituting the dementia inspection system 700 comprises a sentence storing section 711, a color information table storing section 712, a first examination chart producing section 713, a question storing section 714, a second examination chart producing section 715, a chart storing section 717, a chart sending section 716, an answer receiving section 718, a dementia degree inspecting section 720, a dementia degree storing section 719, a result sending section 721, an inspection receiving permission judging section 722, and a sentence range detecting section 723. Among them, the sentence storing section 711, the color information table storing section 712, the first examination chart producing section 713, the question storing section 714, the second examination chart producing section 715, the dementia degree inspecting section 720, the dementia degree storing section 719, the inspection receiving permission judging section 722, and the sentence range detecting section 723 have the same functions as those of the sentence storing section 123, the color information table storing section 124, the first examination chart producing section 125, the question storing section 126, the second examination chart producing section 127, the dementia degree inspecting section 122, the dementia degree storing section 133, the inspection receiving permission judging section 132, and the sentence range detecting section 135 which constitute the dementia degree examining apparatus 120 shown in Fig. 5, described above. Here, redundancy explanation will be omitted. The inspection receiving permission judging section 721 determines whether the request for inspection is permitted or prohibited based on personal information sent from the dementia inspection client 720, and when the request for inspection is prohibited, the inspection receiving permission judging section 721 prohibits the chart sending section 716 from sending the first and second examination charts and first and second pre-test examination charts.

In the dementia inspection server 710, the first examination chart and the second examination chart produced by the first examination chart producing section 713 and the second examination chart producing section 715 are temporarily stored in the chart storing section 716. When the request for inspection is permitted, the chart sending section 717 sends the first and secondpre-test examination charts storedinthe sentence storing section 711, and the first examination chart and the second examination chart stored in the chart storing section 716 to the dementia inspection client 730. The answer receiving section 718 receives answers to the examination chart sent from the dementia inspection client 730. The result sending section 721 sends a dementia degree inspection result obtained by the dementia degree inspecting section 720 to the dementia inspection client 730. In terms of hardware, the communication board (see Fig. 2) of the computer system operated as the dementia inspection server 710 corresponds to these members.

The dementia inspection client 730 constituting the dementia inspection system 700 comprises a chart receiving section 731, a chart display section 732, a start instructing section 733, an answer time control section 734, an answer obtaining section 735, an answer sending section 736, a pre-test executing section 737, a result receiving section 738, and a result display section 739. Among them, the chart display section 732, the start instructing section 733, the answer time control section 734, the answer obtaining section 735, the pre-test executing section 737, and the result display section 739 have the same functions as those of the chart display section 128, the start instructing section 129, the answer time control section 130, the answer obtaining section 121, the pre-test executing section 134, and the result display section 131 in the dementia inspection apparatus 120 shown in Fig. 5, and redundancy explanation will be omitted.

The chart receiving section 731 receives a dementia degree examining chart sent from the dementia inspection server 710 through the line of communication 600, the answer sending section 736 sends answer obtained by the answer obtaining section 734 to the dementia inspection server 710. The result receiving section 738 receives the dementia degree inspection result sent from the dementia inspection server 710. In terms of hardware, the communication board (see Fig. 2) of the computer system operated as the dementia inspection client 730 corresponds to these members.

The procedure for examining the dementia degree is the same as that of the dementia inspection apparatus shown in Fig. 3 except the information is transmitted through the line of communication 600 and thus, explanation thereof will be omitted here.

According to the dementia inspection system 700 shown in Figs. 17 and 18, a subject uses his or her own personal computer connected to the dementia inspection server 710 through the line of communication 600 as the dementia inspection client 730, and the subject can receive the inspection of his or her dementia degree in his or her own home.

In the above-explained embodiments of the dementia inspection apparatus of the present invention, as one example of the sentence range detecting section, a range of the inspection sentence which a subj ect was able to read within the predetermined answer time limit is detected while referring to answers from the subject made with respect to the first examination chart. However, the sentence range detecting section of the present invention is not limited to this. For example, when answering to the first examination chart, a subject may be allowed to click a character at the end of a sentence range which the subject was able to read within the predetermined answer time limit, thereby obtaining information concerning how much the subject was able to read the inspection sentence, and a range of he inspection sentence from the first line to a line including the last character may be defined as the sentence range which the subject was able to read within the predetermined answer time limit. Further, when answering to the first examination chart, a sensor which detects a position of vantage point of a subject moving on the inspection sentence displayed on the display may further be provided, and of positions of vantage point of the subject detected within the predetermined answer time limit, apositionclosest to the sentence end is extracted, a line closest to the extracted position is obtained from lines of the inspection sentence, and a range of the inspection sentence from the first line to the obtained line may be defined as a sentence range which the subj ect was able to read within the predetermined answer time limit.

## Claims

1. A dementia inspection apparatus comprising: an answer obtaining section (121; 718) and a dementia degree inspecting section (122; 719),
wherein the answer obtaining section (121; 718) is configured to obtain answers from a subject made within a predetermined answer time limit to each of a first examination chart and a second examination chart, the first examination chart has inspection sentences in which a character group constituting a story including color words each representing color is tinted with plural colors such that individual color word has characters of the same color, requires a determination as to whether a color of characters constituting the color word is the same color as color represented by the color word, and requires an answer in a style capable of objectively determining whether the determination is correct or error, the second examination chart has a combination of plural questions concerning contents of the inspection sentences and plural answers which are prepared for each question and one of which is to be selected, and
the dementia degree inspection section (122; 719) is configured to determine a dementia degree representing a degree of dementia of the subject based on the answers obtained by the answer obtaining section.

2. The dementia inspection apparatus according to claim 1, further comprising a first examination chart forming section (125; 713), wherein the first examination chart forming section has plural kinds of inspection sentences tinted with colors in different coloring manners and selects one kind of inspection sentences from the plural kinds of inspection sentences as inspection sentences of the first examination chart, thereby forming the first examination chart.

3. The dementia inspection apparatus according to claim 1, further comprising a second examination chart forming section (127; 715), wherein the second examination chart forming section has plural kinds of question units each comprising a combination of a question concerning a story represented by the inspection sentences and plural answers which are prepared for the question and which are to be selected, a predetermined number of question units are selected from the plural kinds of question units, thereby forming the second examination chart.

4. The dementia inspection apparatus according to claim 3, wherein
the plural kinds of question units are associated with positions in the inspection sentences,
the dementia inspection apparatus further comprises a sentence range detecting section (135; 723) which is operable to detect a range of the inspection sentences which a subject was able to read within a predetermined answer time limit with respect to the first examination chart,
the second examination chart forming section is operable to select a question unit associated with a position in the range detected by the sentence range detecting section in the inspection sentence, thereby forming the second examination chart.

5. The dementia inspection apparatus according to claim 1, wherein color words in the character group of the inspection sentences are tinted with plural colors such that individual color word has characters of the same color, characters in the character group except the color words are tinted with a single color.

6. The dementia inspection apparatus according to claim 1, wherein in the inspection sentences, character strings divided by specific kind of characters are tinted with colors different from each other.

7. The dementia inspection apparatus according to claim 1, further comprising:
a chart display section (128) which is operable to display the first examination chart and the second examination chart;
a start instructing section (129) which is operable to instruct the subject to start inputting answers to the first examination chart and the second examination chart; and
an answer time control section (130) which is operable to prohibit the subject from inputting answer when time elapsed after the start instructing section instructed to start inputting answer to the first examination chart reaches a predetermined answer time limit in accordance with the first examination chart, and prohibit the subject from inputting answer when time elapsed after the start instructing section instructed to start inputting answer to the second examination chart reaches a predetermined answer time limit in accordance with the second examination chart.

8. The dementia inspection apparatus according to claim 1, further comprising a result display section (131) which is operable to display a dementia degree inspection result obtained by the dementia degree inspecting section.

9. The dementia inspection apparatus according to claim 1, further comprising an inspection receiving permission judging section (132; 722) which is operable to permit or prohibit a request for inspection of an inspection wisher in accordance with whether or not a predetermined period of time is elapsed after the inspection wisher received inspection last time.

10. The dementia inspection apparatus according to claim 1, further comprising a dementia degree storing section (133; 720) which is operable to store correspondence between answers and dementia degree with respect to both the first examination chart and second examination chart, wherein
the dementia degree inspecting section refers to the dementia degree storing section, and determines a dementia degree of the subject from answers to both the first examination chart and second examination chart of the current subject obtained by the answer obtaining section.

11. The dementia inspection apparatus according to claim 10, wherein from the answers to the first examination chart obtained by the answer obtaining section, the dementia degree inspecting section is operable to extract a number of correct answers as to whether color of characters constituting the color word is the same color as that represented by the color word, a number of error answers erroneously determined, a number of oversights of color words, and a number of erroneous recognition of characters other than the color words, and extract a number of correct meaning grasps with respect to plural questions concerning a story described by the inspection sentences from answers to the second examination chart obtained by the answer obtaining section, and compare these extracted numbers with predetermined reference values with respect to these numbers, thereby determining the dementia degree, and
the dementia degree storing section is operable to store the predetermined reference values.

12. The dementia inspection apparatus according to claim 11, wherein in plural alternatives which are prepared as answers to the questions and one of which is to be selected, the second examination chart includes alternatives for informing that the subject does not know the correct answer, and
the dementia degree inspecting section is further operable to extract a number of unclear answers showing how many alternatives for informing that the subject does not know the correct answer the subject selected from answers to the second examination chart obtained by the answer obtaining section, and determines the dementia degree based on the number of unclear answers, the number of correct answers, the number of error answers, the number of oversights, the number of erroneous recognition, and the number of meaning grasps.

13. The dementia inspection apparatus according to claim 1, further comprising a pre-test executing section (134) which is operable to carry out a pre-test including a pre-test first examination chart and a pre-test second examination chart before the dementia degree is determined using the first examination chart and the second examination chart, the pre-test first examination chart includes pre-test sentences in which a character group constituting a story including color words each representing color is tinted with plural colors such that individual color word has characters of the same color, requires a determination as to whether a color of the characters constituting the color word is the same color as color represented by the color word, and requires an answer in a style capable of objectively determining whether the determination is correct or error, the pre-test second examination chart has a combination of plural questions concerning contents of the pre-test sentences and plural answers which are prepared for each question and one of which is to be selected,
wherein the answer obtaining section is operable to obtain answers of the subject made within predetermined answer time limits to both the pre-test first examination and the pre-test second examination chart, and
wherein the dementia degree inspecting section is operable to determine the dementia degree which represents a degree of dementia of the subject based on the answers to the pre-test first examination chart and the pre-test second examination chart.

## Patentansprüche

1. Demenzuntersuchungsvorrichtung, welche Folgendes umfasst:
einen Antworterhaltabschnitt (121; 718) und einen Demenzgraduntersuchungsabschnitt (122; 719),
wobei der Antworterhaltabschnitt (121; 718) zum Erhalten von Antworten von einem Subjekt, die innerhalb einer vorbestimmten Antwortzeitgrenze auf je eine erste Prüfgrafik und eine zweite Prüfgrafik gegeben werden, konfiguriert ist, wobei die erste Prüfgrafk Untersuchungssätze aufweist, in welchen eine Zeichengruppe, die eine Geschichte darstellt, welche Farbwörter beinhaltet, die jeweils für eine Farbe stehen, mit mehreren Farben eingefärbt ist, derart, dass ein einzelnes Farbwort Zeichen der gleichen Farbe aufweist, eine Bestimmung dessen erfordert, ob eine Farbe von Zeichen, die das Farbwort darstellen, die gleiche Farbe wie die Farbe ist, für die das Farbwort steht, und eine Antwort in einem Stil erfordert, durch den objektiv festgelegt werden kann, ob die Bestimmung korrekt oder falsch ist, und wobei die zweite Prüfgrafik eine Kombination aus mehreren Fragen, welche den Inhalt der Untersuchungssätze betreffen, und mehreren Antworten, welche für jede Frage vorbereitet wurden und von welchen eine ausgewählt werden soll, aufweist, und
wobei der Demenzgraduntersuchungsabschnitt (122; 719) zum Bestimmen eines Demenzgrades, welcher einen Demenzgrad des Subjektes darstellt, auf der Grundlage der Antworten, die durch den Antworterhaftabschnitt erhalten wurden, konfiguriert ist.

2. Demenzuntersuchungsvorrichtung nach Anspruch 1, welche ferner einen ersten Prüfgrafikbildungsabschnitt (125; 713) umfasst, wobei der erste Prüfgrafikbildungsabschnitt mehrere Arten von Untersuchungssätzen eingefärbt mit Farben in unterschiedlichen Farbgebungsmethoden aufweist und eine Art von Untersuchungssätzen aus den mehreren Arten von Untersuchungssätzen als Untersuchungssätze der ersten Prüfgrafik auswählt, wodurch die erste Prüfgrafik gebildet wird.

3. Demenzuntersuchungsvorrichtung nach Anspruch 1, welche ferner einen zweiten Prüfgrafikbildungsabschnitt (127; 715) umfasst, wobei der zweite Prüfgrafikbildungsabschnitt mehrere Arten von Frageeinheiten aufweist, welche jeweils eine Kombination aus einer Frage, welche eine Geschichte betrifft, die durch die Untersuchungssätze dargestellt wird, und mehreren Antworten, welche für die Frage vorbereitet wurden und welche auszuwählen sind, umfassen, wobei eine vorbestimmte Zahl von Frageeinheiten aus den mehreren Arten von Frageeinheiten ausgewählt wird, wodurch die zweite Prüfgrafik gebildet wird.

4. Demenzuntersuchungsvorrichtung nach Anspruch 3, wobei
die mehreren Arten von Frageeinheiten mit Positionen in den Untersuchungssätzen im Zusammenhang stehen,
die Demenzuntersuchungsvorrichtung ferner einen Satzbereicherkennungsabschnitt (135; 723) umfasst, welcher zum Erkennen eines Bereiches der Untersuchungssätze geeignet ist, welche ein Subjekt innerhalb einer vorbestimmten Antwortzeitgrenze in Bezug auf die erste Prüfgrafk lesen konnte,
der zweite Prüfgrafikbildungsabschnitt zum Auswählen einer Frageeinheit im Zusammenhang mit einer Position in dem Bereich, der durch den Satzbereicherkennungsabschnitt in dem Untersuchungssatz erkannt wurde, geeignet ist, wodurch die zweite Prüfgrafik gebildet wird.

5. Demenzuntersuchungsvorrichtung nach Anspruch 1, wobei Farbwörter in der Zeichengruppe der Untersuchungssätze mit mehreren Farben eingefärbt sind, derart, dass ein einzelnes Farbwort Zeichen der gleichen Farbe aufweist, und Zeichen in der Zeichengruppe außer den Farbwörtern mit einer einzelnen Farbe eingefärbt sind.

6. Demenzuntersuchungsvorrichtung nach Anspruch 1, wobei in den Untersuchungssätzen Zeichenketten, die durch eine spezifsche Art von Zeichen geteilt sind, mit Farben eingefärbt sind, die sich voneinander unterscheiden.

7. Demenzuntersuchungsvorrichtung nach Anspruch 1, welche ferner Folgendes umfasst:
einen Grafikanzeigeabschnitt (128), welcher zum Anzeigen der ersten Prüfgrafik und der zweiten Prüfgrafik geeignet ist;
einen Startanweisungsabschnitt (129), welcher zum Anweisen des Subjektes, mit der Eingabe von Antworten auf die erste Prüfgrafik und die zweite Prüfgrafik zu beginnen, geeignet ist; und
einen Antwortzeitkontrollabschnitt (130), welcher zum Verbieten dessen, dass das Subjekt eine Antwort eingibt, wenn eine Zeit, die, nachdem der Startanweisungsabschnitt den Beginn der Eingabe einer Antwort auf die erste Prüfgrafik angewiesen hat, vergangen ist, eine vorbestimmte Antwortzeitgrenze in Übereinstimmung mit der ersten Prüfgrafik erreicht, und zum Verbieten dessen, dass das Subjekt eine Antwort eingibt, wenn eine Zeit, die, nachdem der Startanweisungsabschnitt den Beginn der Eingabe einer Antwort auf die zweite Prüfgrafik angewiesen hat, vergangen ist, eine vorbestimmte Antwortzeitgrenze in Übereinstimmung mit der zweiten Prüfgrafik erreicht, geeignet ist.

8. Demenzuntersuchungsvorrichtung nach Anspruch 1, welche ferner einen Ergebnisanzeigeabschnitt (131) umfasst, welcher zum Anzeigen eines Demenzgraduntersuchungsergebnisses geeignet ist, das durch den Demenzgraduntersuchungsabschnitt erhalten wurde.

9. Demenzuntersuchungsvorrichtung nach Anspruch 1, welche ferner einen Untersuchungserhaltgenehmigung-Beurteilungsabschnitt (132; 722) umfasst, welcher zum Genehmigen oder Verbieten einer Anfrage für die Untersuchung eines Untersuchungsersuchenden in Übereinstimmung damit, ob ein vorbestimmter Zeitraum, nachdem der Untersuchungsersuchende das letzte Mal eine Untersuchung erhalten hat, verstrichen ist oder nicht, geeignet ist.

10. Demenzuntersuchungsvorrichtung nach Anspruch 1, welche ferner einen Demenzgradspeicherabschnitt (133; 720) umfasst, welcher zum Speichern von Übereinstimmung zwischen Antworten und Demenzgrad in Bezug auf sowohl die erste Prüfgrafik als auch die zweite Prüfgrafik geeignet ist, wobei
sich der Demenzgraduntersuchungsabschnitt auf den Demenzgradspeicherabschnitt bezieht und einen Demenzgrad des Subjektes aus Antworten des aktuellen Subjektes sowohl auf die erste Prüfgrafik als auch die zweite Prüfgrafik, die durch den Antworterhaltabschnitt erhalten werden, bestimmt.

11. Demenzuntersuchungsvorrichtung nach Anspruch 10, wobei der Demenzgraduntersuchungsabschnitt, aus den Antworten auf die erste Prüfgrafik, die durch den Antworterhaltabschnitt erhalten werden, zum Extrahieren einer Zahl korrekter Antworten hinsichtlich dessen, ob eine Farbe von Zeichen, welche für das Farbwort stehen, die gleiche Farbe ist, wie die durch das Farbwort dargestellte, einer Zahl falscher Antworten, die fälschlicherweise bestimmt wurden, einer Zahl von Übersehungen von Farbwörtern und einer Zahl falscher Erkennungen von anderen Zeichen als den Farbwörtern und zum Extrahieren einer Zahl von korrekten Bedeutungserfassungen in Bezug auf mehrere Fragen, welche eine Geschichte betreffen, die durch die Untersuchungssätze beschrieben wird, aus Antworten auf die zweite Prüfgrafik, die durch den Antworterhaltabschnitt erhalten werden und zum Vergleichen dieser extrahierten Zahlen mit vorbestimmten Referenzwerten in Bezug auf diese Zahlen geeignet ist, wodurch der Demenzgrad bestimmt wird, und
der Demenzgradspeicherabschnitt zum Speichern der vorbestimmten Referenzwerte geeignet ist.

12. Demenzuntersuchungsvorrichtung nach Anspruch 11, wobei die zweite Prüfgrafik, bei mehreren Alternativen, welche als Antworten auf die Fragen vorbereitet wurden und aus welchen eine auszuwählen ist, Alternativen beinhaltet, um darüber zu informieren, dass das Subjekt die korrekte Antwort nicht weiß, und
der Demenzgraduntersuchungsabschnitt ferner zum Extrahieren einer Zahl von unklaren Antworten, welche zeigen, wie viele Alternativen zum Informieren darüber, dass das Subjekt die korrekte Antwort nicht weiß, das Subjekt aus den Antworten auf die zweite Prüfgrafik, die durch den Antworterhaltabschnitt erhalten werden, ausgewählt hat, geeignet ist und den Demenzgrad auf der Grundlage der Zahl unklarer Antworten, der Zahl korrekter Antworten, der Zahl falscher Antworten, der Zahl von Übersehungen, der Zahl von falschen Erkennungen und der Zahl von Bedeutungserfassungen bestimmt.

13. Demenzuntersuchungsvorrichtung nach Anspruch 1, welche ferner einen Vortestausführungsabschnitt (134) umfasst, welcher zum Ausführen eines Vortests, welcher eine erste Vortest-Prüfgrafik und eine zweite Vortest-Prüfgrafik beinhaltet, bevor der Demenzgrad unter Verwendung der ersten Prüfgrafik und der zweiten Prüfgrafik bestimmt wird, geeignet ist, wobei die erste Vortest-Prüfgrafik Vortest-Sätze beinhaltet, in welchen eine Zeichengruppe, welche eine Geschichte darstellt, die Farbwörter beinhaltet, welche jeweils für eine Farbe stehen, mit mehreren Farben eingefärbt ist, derart, dass ein einzelnes Farbwort Zeichen der gleichen Farbe aufweist, eine Bestimmung dessen erfordert, ob eine Farbe der Zeichen, welche für das Farbwort stehen, die gleiche Farbe wie die Farbe ist, für die das Farbwort steht, und eine Antwort in einem Stil erfordert, durch den objektiv festgelegt werden kann, ob die Bestimmung korrekt oder falsch ist, und wobei die zweite Vortest-Prüfgrafik eine Kombination aus mehreren Fragen, die den Inhalt der Vortest-Sätze betreffen, und mehreren Antworten, welche für jede Frage vorbereitet wurden und von welchen eine auszuwählen ist, aufweist,
wobei der Antworterhaltabschnitt zum Erhalten von Antworten des Subjektes, die innerhalb vorbestimmter Antwortzeitgrenzen sowohl auf die erste Vortest-Prüfgrafik als auch auf die zweite Vortest-Prüfgrafik gegeben werden, geeignet ist, und
wobei der Demenzgraduntersuchungsabschnitt zum Bestimmen des Demenzgrades, welcher einen Demenzgrad des Subjektes darstellt, auf der Grundlage der Antworten auf die erste Vortest-Prüfgrafik und die zweite Vortest-Prüfgrafik geeignet ist.

## Revendications

1. Dispositif de vérification de la démence comprenant une section de réception de réponses (121 ; 718) et une section de vérification du degré de la démence (122 ; 719),
**caractérisé en ce que** la section de réception de réponses (121 ; 718) est configurée pour recevoir des réponses d'une personne dans une limite prédéterminée de temps de réponse à chacune d'un premier tableau d'examen et d'un second tableau d'examen, le premier tableau d'examen ayant des phrases de vérification dans lesquelles un groupe de caractères, constituant une histoire avec des mots de couleurs dont chacun représente une couleur, est teinté avec plusieurs couleurs de façon qu'un mot de couleur individuel comprend des caractères de même couleur, requiert une décision de savoir si une couleur de caractères constituant le mot de couleur est la même couleur que la couleur représentée par le mot de couleur, et requiert une réponse dans un style capable de déterminer objectivement si la décision est correcte ou fausse, le second tableau d'examen comprenant une combinaison de plusieurs questions relatives à des contenus des phrases de vérification et de plusieurs réponses qui sont préparées pour chaque question et dont il faut choisir une, et la section de vérification du degré de la démence (122 ; 719) est configurée pour déterminer un degré de démence représentant un degré de la démence de la personne sur la base des réponses obtenues par la section de réception de réponses.

2. Dispositif de vérification de la démence selon la revendication 1, comprenant en outre une section (125 ; 713) formant un premier tableau d'examen, où la section formant un premier tableau d'examen comporte une pluralité de phrases de vérification teintées avec des couleurs en différentes façons de coloration et choisit un type de phrases de vérification de la pluralité de types de phrases de vérification comme phrases de vérification du premier tableau d'examen, formant ainsi le premier tableau d'examen.

3. Dispositif de vérification de la démence selon la revendication 1, comprenant en outre une section (127 ; 715) formant un second tableau d'examen, où la section formant un second tableau d'examen comporte une pluralité de types d'unités de questions, chacune comprenant une combinaison d'une question relative à une histoire représentée par les phrases de vérification et de plusieurs réponses qui sont préparées pour la question et qui sont à choisir, où un nombre prédéterminé d'unités de questions est choisi parmi la pluralité de types d'unités de questions, formant ainsi le second tableau d'examen.

4. Dispositif de vérification de la démence selon la revendication 3, **caractérisé en ce que**
la pluralité de types d'unités de questions est associée à des positions dans les phrases de vérification,
le dispositif de vérification de la démence comprend en outre une section (135 ; 723) de détection de la longueur de la phrase qui est apte à détecter une longueur des phrases de vérification qu'une personne était capable de lire dans une limite de temps de réponse prédéterminée par rapport au premier tableau d'examen,
la section formant un second tableau d'examen est apte à choisir une unité de questions associée à une position sur la longueur détectée par la section de détection de la longueur dans la phrase de vérification, formant ainsi le second tableau d'examen.

5. Dispositif de vérification de la démence selon la revendication 1, **caractérisé en ce que** des mots de couleur dans le groupe de caractères des phrases de vérification sont teintés avec plusieurs couleurs de façon qu'un mot de couleur individuel comporte des caractères de même couleur, des caractères dans le groupe de caractères à l'exception des mots de couleur, sont teintés avec une seule couleur.

6. Dispositif de vérification de la démence selon la revendication 1, **caractérisé en ce que** dans les phrases de vérification, des chaînes de caractères divisées par un type spécifique de caractères sont teintées avec des couleurs différentes les unes des autres.

7. Dispositif de vérification de la démence selon la revendication 1, comprenant en outre
une section d'affichage de tableau (128) qui est apte à afficher le premier tableau d'examen et le second tableau d'examen,
une section (129) donnant un ordre de démarrage qui est apte à donner à la personne l'ordre de commencer à rentrer des réponses au premier tableau d'examen et au second tableau d'examen et
une section (130) de contrôle du temps de réponse qui est apte à empêcher la personne de rentrer une réponse lorsque le temps écoulé après que la section donnant un ordre de démarrage a donné l'ordre à la personne de commencer à rentrer des réponses au premier tableau d'examen atteint une limite de temps de réponse prédéterminée par rapport au premier tableau d'examen, et à empêcher la personne de rentrer une réponse lorsque le temps écoulé après que la section donnant un ordre de démarrage a donné l'ordre à la personne de commencer à rentrer des réponses au second tableau d'examen atteint une limite de temps de réponse prédéterminée par rapport au second tableau d'examen.

8. Dispositif de vérification de la démence selon la revendication 1, comprenant en outre une section d'affichage de résultat (131) qui est apte à afficher un résultat de vérification du degré de la démence obtenu par la section de vérification du degré de la démence.

9. Dispositif de vérification de la démence selon la revendication 1, comprenant en outre une section (132 ; 722) d'évaluation de l'autorisation de recevoir une vérification qui est apte à permettre ou à interdire une demande de vérification d'un demandeur de vérification selon qu'un lapse de temps prédéterminé s'est écoulé ou non après que le demandeur de vérification a reçu une vérification la dernière fois.

10. Dispositif de vérification de la démence selon la revendication 1, comprenant en outre une section de stockage de degrés de la démence (133 ; 720) qui est apte à stocker une correspondance entre des réponses et des degrés de la démence aussi bien par rapport au premier tableau d'examen qu'au second tableau d'examen,
où la section de vérification du degré de la démence se réfère à la section de stockage de degrés de la démence et détermine un degré de la démence de la personne à partir des réponses aussi bien au premier tableau d'examen qu'au second tableau d'examen de la personne actuelle obtenues par la section de réception de réponses.

11. Dispositif de vérification de la démence selon la revendication 10, **caractérisé en ce qu'**à partir des réponses au premier tableau d'examen obtenues par la section de réception de réponses, la section de vérification du degré de la démence est apte à extraire un nombre de réponses correctes aux questions de savoir si la couleur des caractères constituant le mot de couleur est la même couleur que celle représentée par le mot de couleur, un nombre de réponses fausses déterminées par erreur, un nombre de mots de couleurs non aperçus, et un nombre de reconnaissances erronées de caractères autres que les mots de couleurs, et à extraire un nombre de compréhensions correctes de signification par rapport à la pluralité de questions concernant une histoire décrite par les phrases de vérification, des réponses au second tableau d'examen obtenues par la section de réception de réponses, et à comparer ces nombres extraits à des valeurs de référence prédéterminées relatives à ces nombres, déterminant ainsi le degré de la démence, et
que la section de stockage de degrés de la démence est apte à stocker les valeurs de référence prédéterminées.

12. Dispositif de vérification de la démence selon la revendication 11, **caractérisé en ce que** dans les alternatives nombreuses qui sont préparées comme réponses aux questions et dont une est à choisir, le second tableau d'examen comporte des alternatives pour informer que la personne ne connaît pas la réponse correcte et
que la section de vérification du degré de la démence est apte, en plus, à extraire un nombre de réponses non claires montrant combien d'alternatives pour informer que la personne ne connaît pas la réponse correcte, la personne a choisi parmi les réponses au second tableau d'examen obtenues par la section de réception des réponses, et détermine le degré de la démence sur la base du nombre de réponses non claires, le nombre de réponses correctes, le nombre de réponses erronées, le nombre d'oubli, le nombre de reconnaissances erronées et le nombre de compréhensions de la signification.

13. Dispositif de vérification de la démence selon la revendication 1, comprenant en outre une section de mise en oeuvre d'une pré-évaluation (134) qui est apte à effectuer une pré-évaluation comprenant un premier tableau de pré-évaluation et un second tableau de pré-évaluation avant que le degré de la démence ne soit déterminé en utilisant le premier tableau d'examen et le second tableau d'examen, le premier tableau de pré-évaluation comprenant des phrases de pré-évaluation dans lesquelles un groupe de caractères, constituant une histoire avec des mots de couleurs dont chacun représente une couleur, est teinté avec plusieurs couleurs de façon qu'un mot de couleur individuel comprend des caractères de même couleur, requiert une décision de savoir si une couleur de caractères constituant le mot de couleur est la même couleur que la couleur représentée par le mot de couleur, et requiert une réponse dans un style capable de déterminer objectivement si la décision est correcte ou fausse, le second tableau de pré-évaluation comprenant une combinaison de plusieurs questions relatives à des contenus des phrases de pré-évaluation et de plusieurs réponses qui sont préparées pour chaque question et dont il faut choisir une,
la section de réception de réponses (121 ; 718) étant apte à recevoir des réponses de la personne données dans une limite prédéterminée de temps de réponse à chacune d'un premier tableau de pré-évaluation et d'un second tableau de pré-évaluation, et
la section de vérification du degré de la démence est apte à déterminer le degré de démence représentant un degré de la démence de la personne sur la base des réponses au premier tableau de pré-évaluation et au second tableau de pré-évaluation.
